# EUROPEAN PATENT APPLICATION

(11) **EP 4 431 532 A2**
(43) Date of publication of application: **18.09.2024**
(21) Application number: 24192851.4
(22) Date of filing: 05.03.2020
(51) Int. Cl.: C08F 2/26

(54) **REACTIVE SURFACTANTS**

(30) Priority: 08.03.2019 US 201962815555 P
(62) Divisional of application: 20714824.8
(71) Applicant: Stepan Company, Northfield, Illinois 60093 (US)
(72) Inventor: BOEBEL, Timothy A., Wilmette, IL, 60091 (US); FARRIS, Samantha Michelle, Chicago, IL, 60645 (US); LUEBKE, Gary, Gyrnee, IL, 60031 (US); ROJAS, Carolina E., Highland Park, IL, 60035 (US); SPAULDING, Chris, Evanston, IL, 60201 (US); TERRY, Michael R., Gumee, IL, 60031 (US); WOLFE, Patrick Shane, Palatine, IL, 60074 (US); ZAUG, Julia, Evanston, IL, 60201 (US)
(74) Representative: Prinz & Partner mbB

(57) **Abstract**

Processes for making reactive surfactants are disclosed. In one such process, an alkyl- or alkenylsuccinic anhydride is reacted with an olefin-functional nucleophile to produce an olefin-functional succinate monoester. Surfactant compositions comprising the reactive surfactants made by these processes are also described. The invention includes polymerizable mixtures comprising an acrylic monomer and the surfactant compositions as well as aqueous acrylic latex emulsions and coatings produced from the emulsions. The reactive surfactants deliver stable latex emulsions with reduced tendency for surfactant migration or excessive foaming. Coatings from the emulsions have improved wet adhesion, scrub resistance, and water resistance.

## Description

### FIELD OF THE INVENTION

The invention relates to surfactants having a polymerizable carbon-carbon double bond and their use for making acrylic latex resins and coatings.

### BACKGROUND OF THE INVENTION

Conventional surfactants used for making acrylic latex emulsions for water-based coatings usually lack reactivity with acrylic monomers. Consequently, the resulting latexes can be less stable than desirable. Additionally, the surfactant can migrate within the polymer matrix, which results in formation of hydrophilic domains at the coating surface. These domains render the coating susceptible to adhesion loss, surface damage, and discoloration caused by water penetration.

Reactive surfactants can overcome some of these deficiencies of conventional surfactants. Inclusion of an olefinic unsaturation in a surfactant molecule allows the surfactant to be covalently incorporated into an acrylic latex thereby stabilizing the latex and reducing the tendency of the surfactant to migrate. For some examples of surfactants modified to include polymerizable unsaturation, see U.S. Pat. Nos. 9,051,341; 9,376,510; and 9,637,563.

Imidazolidinone-functional monomers have been used as reactants for improving wet-adhesion properties of aqueous latex coatings (see, e.g., U.S. Pat. Nos. 4,111,877; 4,599,417; 4,426,503; 4,429,095; and 4,632,957). U.S. Pat No. 5,746,946 describes a reaction product of dodecenyl succinic anhydride and 2-aminoethyl imidazolidinone as a corrosion inhibitor for a water-borne alkyd coating; however, use of this material as a reactive surfactant with monomers used to make a latex is not described.

The industry would benefit from the availability of new reactive surfactants. Valuable products would enhance the hydrophobicity of latex coatings made using the surfactants and would improve their water resistance. Coatings with improved adhesion and wet-scrub resistance are also needed. Ideally, surfactants having reduced levels of non-reactive components could be produced easily from readily available reactants and well-established chemistry.

### SUMMARY OF THE INVENTION

In one aspect, the invention relates to a process for making a reactive surfactant. The process comprises, in a first step, reacting a fatty epoxide, a glycidyl ether, or a combination thereof with an olefin-functional nucleophile to produce an olefin-functional hydrophobe. The olefin-functional hydrophobe is reacted with ethylene oxide, propylene oxide, butylene oxides, or a combination thereof to produce an alkoxylate. Optionally, the alkoxylate is converted to the corresponding sulfate, phosphate, or maleate.

In another aspect, the invention relates to a process for making a reactive surfactant. This process comprises first reacting a phenol, polyphenol, alcohol, polyalcohol, or thiol with a fatty epoxide, a first glycidyl ether, or a combination thereof to produce a hydroxy-functional intermediate. The intermediate is then reacted with an olefin-functional glycidyl ether to produce an olefin-functional hydrophobe. In a next step, the olefin-functional hydrophobe is reacted with ethylene oxide, propylene oxide, butylene oxides, or a combination thereof to produce an alkoxylate. Optionally, the alkoxylate is converted to the corresponding sulfate, phosphate, or maleate.

In yet another process, a reactive surfactant is made by a process comprising reacting an alkyl- or alkenylsuccinic anhydride with an olefin-functional nucleophile to produce an olefin-functional succinate monoester, and optionally, neutralizing the resulting monoester.

The invention includes surfactant compositions comprising the reactive surfactants made by the processes described above as well as surfactant compositions having particular structural features as described further hereinbelow.

In other aspects, the invention includes polymerizable mixtures comprising an acrylic monomer and the surfactant compositions described above as well as aqueous acrylic latex emulsions and coatings produced from the latex emulsions.

The inventive reactive surfactants deliver stable latex emulsions with reduced tendency for surfactant migration or excessive foaming. Coatings from the emulsions have improved wet adhesion, scrub resistance, and water resistance. The reduced tendency of the inventive reactive surfactants to migrate and their reduced levels of non-reactive components should translate into coatings having fewer surface defects when compared with commercially available reactive surfactants.

### DETAILED DESCRIPTION OF THE INVENTION

In one aspect, the invention relates to a process for making a reactive surfactant. The process first comprises reacting a fatty epoxide, a glycidyl ether, or a combination thereof with an olefin-functional nucleophile to produce an olefin-functional hydrophobe. The olefin-functional hydrophobe is then reacted with ethylene oxide, propylene oxide, butylene oxides, or a combination thereof to produce an alkoxylate. Optionally, the alkoxylate is converted to the corresponding sulfate, phosphate, or maleate.

### A. Olefin-functional hydrophobe

The olefin-functional hydrophobe is made by reacting a fatty epoxide, a glycidyl ether, or a combination thereof with an olefin-functional nucleophile.

### 1. Fatty epoxide or glycidyl ether

Suitable fatty epoxides have six or more carbons and at least one epoxide group. In some aspects, the fatty epoxide is a C₈-C₂₂ fatty epoxide. In particular aspects, the fatty epoxide is selected from 1,2-epoxyhexane, 1,2-epoxyoctane, 1,2-epoxydecane, 1,2-epoxydodecane, 1,2-epoxytetradecane, 1,2-epoxhexadecane, 1,2-epoxyoctadecane, 1,2-epoxy-7-octene, 1,2-epoxy-9-decene, 1,2-epoxycyclododecane, 4-vinylcyclohexene oxide, 1,2-epoxypolybutenes, and the like, and mixtures thereof.

Suitable glycidyl ethers have one or more glycidyl ether units, typically from 1 to 3 or from 1 to 2 glycidyl ether units. In particular aspects, the glycidyl ether is selected from phenyl glycidyl ether (PGE), n-butyl glycidyl ether, isopropyl glycidyl ether, t-butyl glycidyl ether, 2-ethylhexyl glycidyl ether, allyl glycidyl ether (AGE), cyclohexyl glycidyl ether, benzyl glycidyl ether, guaiacol glycidyl ether, 1-ethoxyethyl glycidyl ether, 2-ethoxyethyl glycidyl ether, 2-methylphenyl glycidyl ether, 2-biphenyl glycidyl ether, monostyrylphenol glycidyl ether, distyrylphenol glycidyl ether, tristyrylphenol glycidyl ether, 3-glycidyl(oxypropyl)trimethoxysilane, 3-glycidyl(oxypropyl)triethoxysilane, propargyl glycidyl ether, resorcinol diglycidyl ether, bisphenol A diglycidyl ether, 1,4-butanediol diglycidyl ether, decyl glycidyl ether, dodecyl glycidyl ether, tetradecyl glycidyl ether, hexadecyl glycidyl ether, octadecyl glycidyl ether, and the like, and mixtures thereof.

Generally, the olefin-functional phenol, polyphenol, alcohol or polyalcohol is reacted with one or more molar equivalents, preferably 1 to 5 molar equivalents or 1 to 3 molar equivalents, or about 1 molar equivalent, of the fatty epoxide or glycidyl ether.

### 2. Olefin-functional nucleophile

An olefin-functional nucleophile is reacted with the fatty epoxide or glycidyl ether to produce the olefin-functional hydrophobe.

### a. Olefin-functional phenol, polyphenol, alcohol, or polyalcohol

In some aspects, the olefin-functional nucleophile is an olefin-functional phenol, an olefin-functional polyphenol, an olefin-functional alcohol, an olefin-functional polyalcohol, an alkoxylate thereof, or a mixture thereof.

Suitable olefin-functional phenols and polyphenols include allyl-, propenyl-, butenyl-, and vinyl-substituted phenols and polyphenols. Examples include allyl-, propenyl-, butenyl-, and vinyl-substituted phenols, bisphenols, catechols, and resorcinols. Suitable olefin-functional phenols include, for example, eugenol, isoeugenol, propenyl guaethol, *trans*-ferulic acid, *trans*-ferulic acid ethyl ester, and *trans*-ferulic acid methyl ester. Suitable olefin-functional phenols include hydroxyl-functional stilbenes such as pterostilbene, *cis*-resveratrol, *trans*-resveratrol, piceatannol, rhapontigenin, isorhapontigenin, and the like, dehydrodimers thereof (e.g., δ-viniferin, ε-viniferin), and mixtures thereof. Other suitable olefin-functional phenols include olefin-substituted styrenated phenols such as olefin-substituted mono- and distyrylphenols. Olefin-substituted styrenated phenols are conveniently prepared in two steps from the corresponding styrenated phenols as described in WO 2018/179913. Thus, in some aspects, the phenol is first converted to an allyl ether (e.g., with an allyl halide), and the ether is subsequently heated to induce Claisen rearrangement to give an allyl- or propenyl-substituted styrenated phenol. In some aspects, a mixture containing mostly a monostyrylphenol (e.g., a 3:1 mixture of mono- and distyrylphenols) is converted to the allyl ether in the first step.

Suitable olefin-functional alcohols and polyalcohols include primary, secondary, or tertiary alcohols having one or more olefin functionalities. Examples include allyl alcohol, methallyl alcohol, ethylene glycol monoallyl ether, cinnamyl alcohol, 2-hydroxy-2-methyl-3-butene, 2-methyl-3-buten-1-ol, 1,4-butanediol monoallyl ether, 1,4-butanediol monovinyl ether, trimethylolpropane diallyl ether, trimethylolpropane allyl ether, triethylolpropane diallyl ether, triethylolpropane allyl ether, cis-3-hexenyl lactate, glycerol α,α'-diallyl ether, 2,7-octadien-1-ol, farnesol, phytol, and the like, alkoxylates thereof, and mixtures thereof. Also suitable are hydroxyl-functionalized norbornene derivatives such as 5-norbornene-2-methanol.

In some aspects, the olefin-functional alcohol or polyalcohol is a reaction product of an olefin-substituted phenol or polyphenol and a glycidyl ether. For instance, an olefin-functional secondary alcohol results from the reaction of an allyl- or propenyl-substituted styrenated phenol with one or more molar equivalents of phenyl glycidyl ether. In another example, an olefin-functional polyalcohol is made by reacting resorcinol with two or more molar equivalents of allyl glycidyl ether.

In some aspects, the olefin-functional nucleophile is an alkoxylate, especially an ethoxylate, of an olefin-functional phenol, an olefin-functional polyphenol, an olefin-functional alcohol, or an olefin-functional polyalcohol. Suitable alkoxylates have one or more alkylene oxide recurring units, especially from ethylene oxide, propylene oxide, butylene oxides, and combinations thereof, which may be in block or random configuration.

### b. Hydroxy-functional acrylates, acrylamides, and alkoxylates

In some aspects, the olefin-functional nucleophile is a hydroxy-functional acrylate, a hydroxy-functional acrylamide, an alkoxylate thereof, or a mixture thereof.

Suitable hydroxy-functional acrylate and acrylamides are well known. In some aspects, the hydroxy-functional acrylate or acrylamide is selected from 2-hydroxyethyl acrylate, 2-hydroxypropyl acrylate, 2-hydroxyethyl methacrylate, 2-hydroxypropyl methacrylate, 2-hydroxyethyl acrylamide, 2-hydroxypropyl acrylamide, 2-hydroxyethyl methacrylamide, 2-hydroxypropyl methacrylamide, 3-(acryloyloxy)-2-hydroxypropyl methacrylate, 2-hydroxy-3-phenoxypropyl acrylate, alkoxylates thereof, and mixtures thereof.

### 3. Exemplary hydrophobes

The representative structures below show hydrophobes that are reaction products of a fatty epoxide or glycidyl ether with an olefin-functional nucleophile. The shorthand names include the names of the reactants used to make the hydrophobes. For instance, in the first example listed, reaction of equimolar amounts of 1,2-epoxytetradecane and 2-allylphenol provides "2-allylphenol-[1,2-epoxytetradecane(1)]." The nomenclature convention used in this application reflects the *starting materials* used. The original 2-allyl group in many cases rearranges partially or completely under the reaction conditions to give the more thermodynamically stable 2-propenyl group.

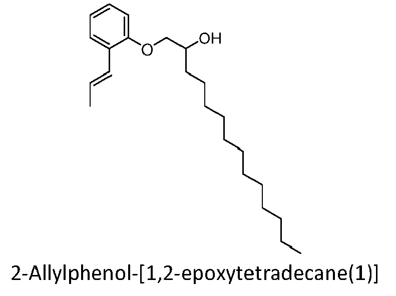

Additional olefin functionality can be introduced in subsequent steps, as in this example wherein 2-allylphenol-[1,2-epoxytetradecane(1)] is further reacted with allyl glycidyl ether:

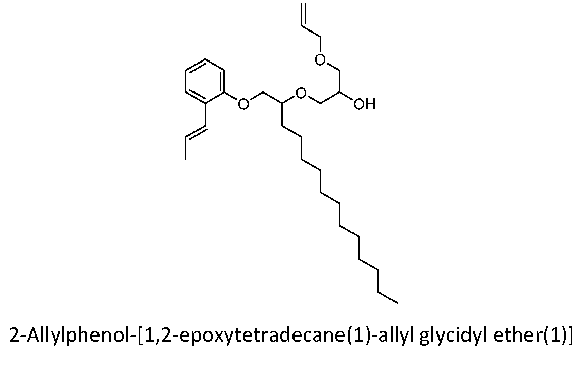

Eugenol is a suitable olefin-functional phenol that can be reacted with a glycidyl ether such as tristyrylphenol glycidyl ether ("TSPGE") or a fatty epoxide to give the hydrophobe:

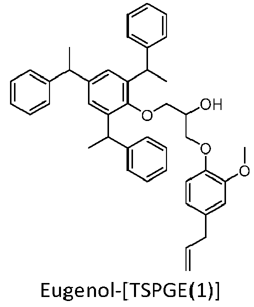

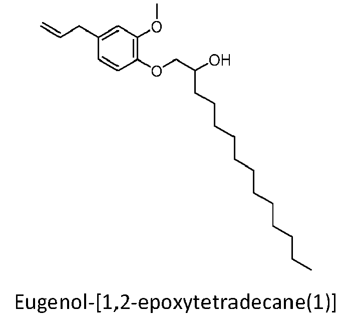

In this example, the olefin-functional nucleophile is the initial reaction product of resorcinol and two molar equivalents of allyl glycidyl ether:

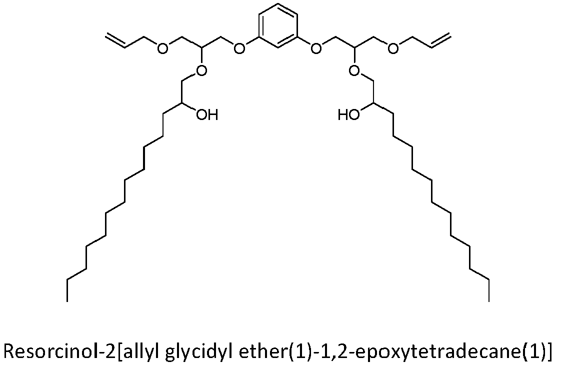

The glycidyl ether reactant can be polyfunctional, as in these examples with resorcinol diglycidyl ether: 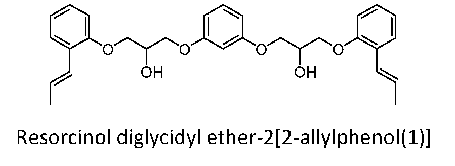 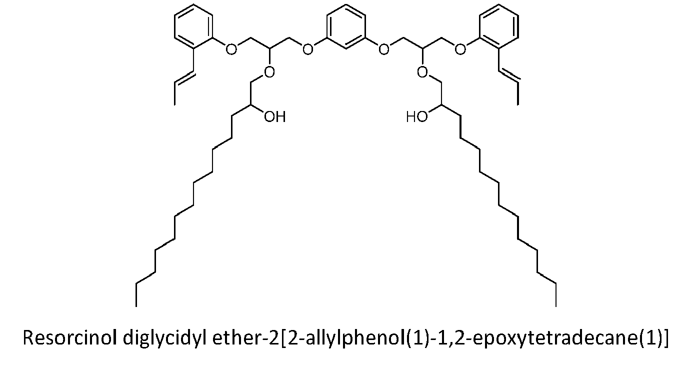 In some cases, olefin functionalities can be appended to an aromatic ring, e.g., the propenyl group(s) shown in these examples with an olefin-functional phenol as a reactant. The exact location of the olefin group(s) in the structures below is uncertain and could be a mixture. As shown earlier, and in the latter pair of examples below, additional olefin functionality can be introduced in subsequent steps.

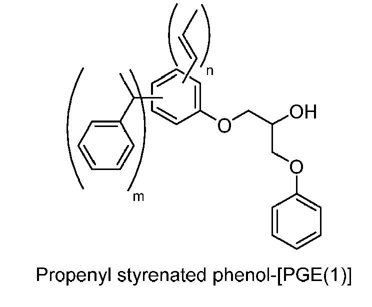

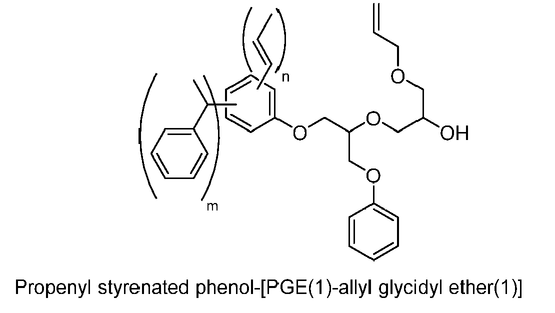

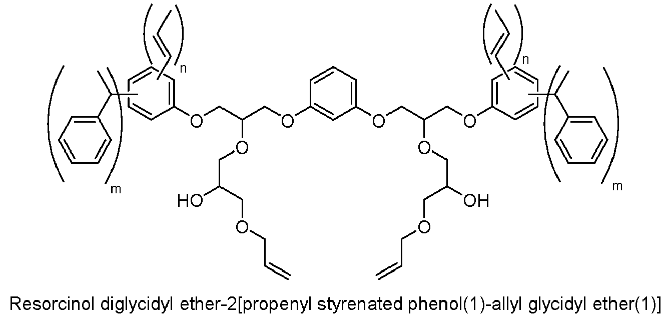

Cinnamyl alcohol, 2-hydroxy-2-methyl-3-butene, 1,4-butanediol vinyl ether, 2,7-octadien-1-ol, or phytol can be used as the olefin-functional nucleophile for making the olefin-functional hydrophobe as shown in these examples:

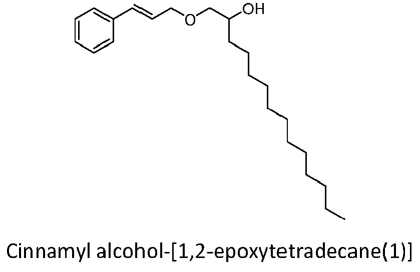

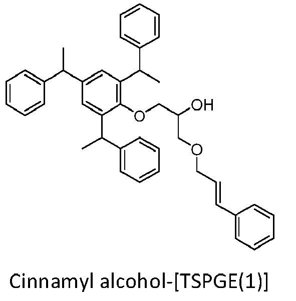

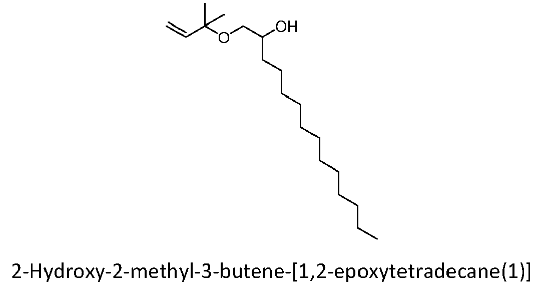

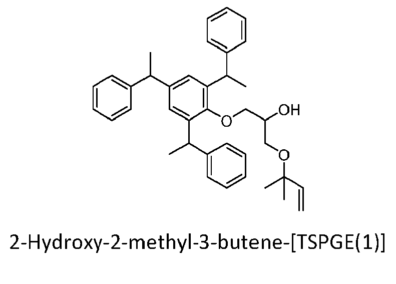

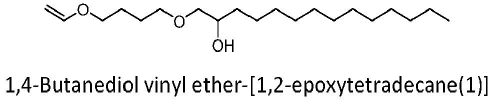

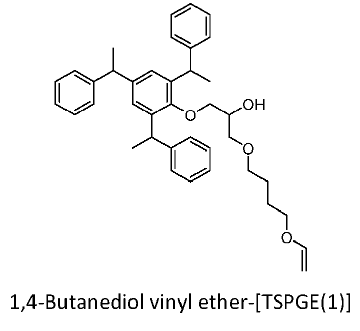

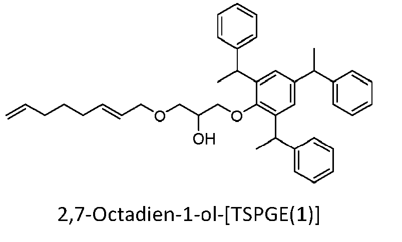

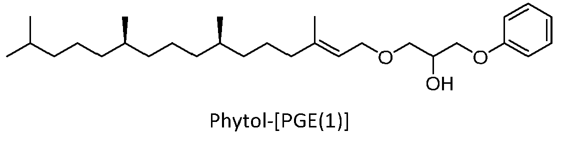

Suitable olefin-functional polyalcohols include trimethylolpropane mono- or diallyl ethers, as illustrated in these hydrophobes:

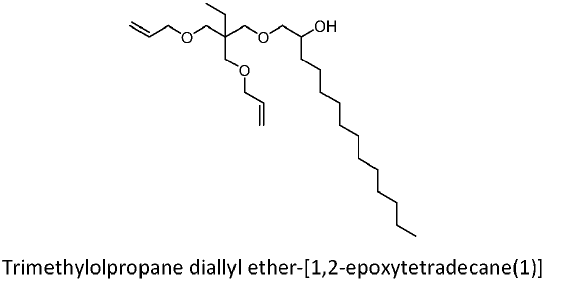

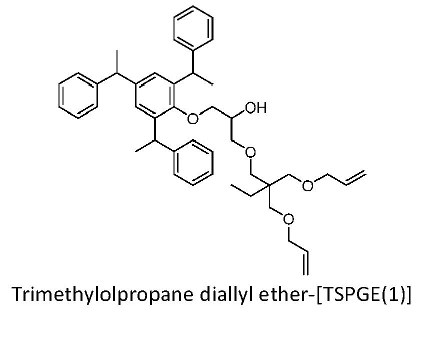

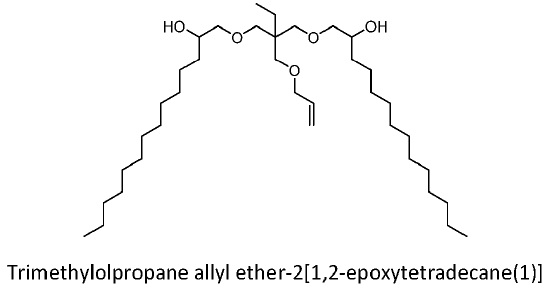

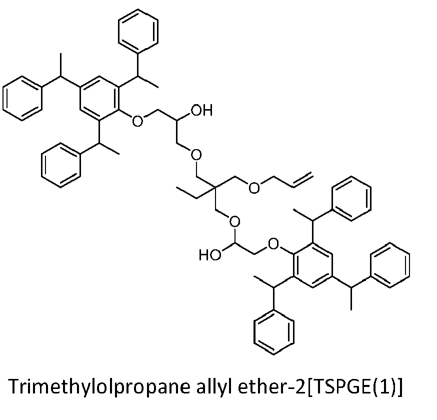

These examples illustrate the use of a hydroxy-functional acrylate as the olefin-functional nucleophile in preparing the olefin-functional hydrophobe:

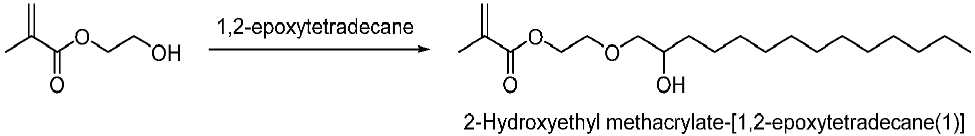

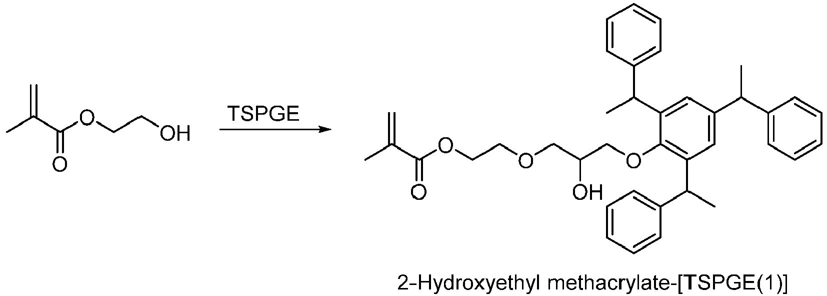

### B. Alkoxylates

The olefin-functional hydrophobes have hydroxyl functionality. Conversion to nonionic or anionic surfactants involves an initial step of reacting the hydrophobes with one or more alkylene oxides to produce alkoxylates. Thus, in one aspect, the olefin-functional hydrophobe is reacted with from 1 to 100 recurring units per hydroxyl equivalent of the hydrophobe of one or more alkylene oxides (AO) selected from ethylene oxide, propylene oxide, butylene oxides, and combinations thereof to give a polymer that is useful by itself as a nonionic surfactant or can be further modified to give an anionic surfactant.

Hydroxyl groups of the hydrophobe react in the presence of a catalyst with one or more equivalents of an alkylene oxide to give the alkoxylated product. In some aspects, enough alkylene oxide is added to introduce 1 to 100, 2 to 50, or 2 to 10 recurring units of alkylene oxide per hydroxyl equivalent of the hydrophobe. In some aspects, the alkylene oxide is selected from ethylene oxide, propylene oxide, butylene oxides, and combinations thereof. The alkylene oxide recurring units can be arranged in random, block, or gradient fashion, e.g., as blocks of a single alkylene oxide, blocks of two or more alkylene oxides (e.g., a block of EO units and a block of PO units), or as a random copolymer. In some aspects, the alkylene oxide is ethylene oxide, propylene oxide, or combinations thereof. In other aspects, the alkylene oxide consists essentially of ethylene oxide.

The alkoxylation reaction is conveniently practiced by gradual addition of the alkylene oxide as mixtures or in steps to produce the desired architecture. The reaction mixture will normally be heated until most or all of the alkylene oxide has reacted to give the desired polymer.

Although basic catalysts are usually most convenient, alternative catalysts can be used in some aspects. For instance, Lewis acids such as boron trifluoride can be used to polymerize alkylene oxides. Double metal cyanide catalysts can also be used (see, e.g., U.S. Pat. Nos. 5,470,813; 5,482,908; 6,852,664; 7,169,956; 9,221,947; 9,605,111; and U.S. Publ. Nos. 2017/0088667 and 2017/0081469).

Following alkoxylation, the polymer can be neutralized to give a nonionic surfactant. In some cases, it may be desirable to convert the hydroxyl groups to other functional groups such as sulfates, phosphates, or maleates, as is described further below. In other cases, it may be desirable to cap the hydroxyl groups to give ethers, esters, carbonates, carbamates, succinates, carbamimidic esters, borates, phosphatidylcholines, ether acids, ester alcohols, ester acids, ether diacids, ether amines, ether ammoniums, ether amides, ether sulfonates, ether betaines, ether sulfobetaines, ether phosphonates, phospholanes, phospholane oxides, or the like, or combinations thereof, using one or more of a variety of available capping groups.

Alkoxylates that are simply neutralized without further modification by capping are useful as reactive nonionic surfactants. In some aspects, these surfactants can be combined with other unsaturated monomers (e.g., acrylic monomers) to produce water- or solvent-based paints and coatings having improved properties, including better water resistance, better wet-scrub resistance, and/or better adhesion properties compared with similar paints and coatings prepared in the absence of a reactive surfactant.

### C. Sulfates, phosphates, and maleates

Optionally, the alkoxylates are converted to sulfates, phosphates, maleates, or salts thereof, to provide anionic surfactants.

Sulfates are conveniently made by reacting the alkoxylate with a suitable sulfating agent such as sulfur trioxide, sulfamic acid, fuming sulfuric acid, chlorosulfonic acid, or the like, according to well-known methods (see, e.g., U.S. Pat. Nos. 2,647,913; 3,931,271; 3,413,331; 3,755,407; and 9,695,385, the teachings of which are incorporated herein by reference). Neutralization with an alkali metal hydroxide, ammonia, or an amine provides a sulfate salt, which has utility as a reactive anionic surfactant.

Phosphates (also called "phosphate esters") are conveniently made by reacting the alkoxylate with a suitable phosphating agent such as P₂O₅, PCl₃, POCl₃, phosphoric acid, polyphosphoric acid, or the like, especially P₂O₅ or polyphosphoric acid, according to well-known methods (see, e.g., U.S. Pat. Nos. 3,346,670; 4,313,847; 4,350,645; and 6,566,408, the teachings of which are incorporated herein by reference). Neutralization of acidic hydrogens with an alkali metal hydroxide, ammonia, or an amine provides a phosphate salt, which has utility as a reactive anionic surfactant. The phosphate is commonly generated as a mixture of mono- and dialkoxyphosphates.

Maleates are conveniently made by reacting the alkoxylate with maleic anhydride or maleic acid, preferably maleic anhydride, followed by neutralization if needed according to well-known methods such as those described in U.S. Pat. Nos. 4,263,413 and 4,532,297, the teachings of which are incorporated herein by reference.

The invention includes an alternative process for making a reactive surfactant. This process comprises first reacting a phenol, polyphenol, alcohol, polyalcohol, or thiol with a fatty epoxide, a first glycidyl ether, or a combination thereof (all as described previously) to produce a hydroxy-functional intermediate. The hydroxy-functional intermediate is then reacted with an olefin-functional glycidyl ether, preferably allyl glycidyl ether, to produce an olefin-functional hydrophobe. Next, the olefin-functional hydrophobe is reacted with ethylene oxide, propylene oxide, butylene oxides, or a combination thereof as described previously to produce an alkoxylate. Optionally, the alkoxylate is converted to the corresponding sulfate, phosphate, or maleate, also as previously described.

In a particular aspect, the phenol is a mono-, di-, or tristyrylphenol, the first glycidyl ether is phenyl glycidyl ether, and the olefin-functional glycidyl ether is allyl glycidyl ether. This provides an olefin-functional hydrophobe having the structure shown below:

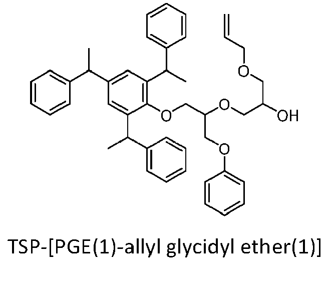

In another particular use of the alternative process, the mono-, di-, or tristyrylphenol is first reacted with a fatty epoxide (here, 1,2-epoxytetradecane), followed by reaction with the olefin-functional glycidyl ether (here, allyl glycidyl ether), as illustrated by this olefin-functional hydrophobe:

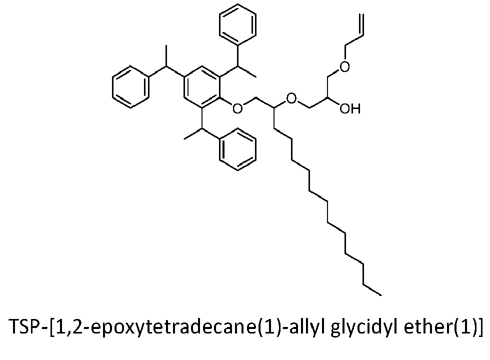

Another hydrophobe example illustrates the use of a thiol reactant (1-dodecanethiol) with the first glycidyl ether (resorcinol diglycidyl ether), prior to a reaction with the olefin-functional glycidyl ether (allyl glycidyl ether):

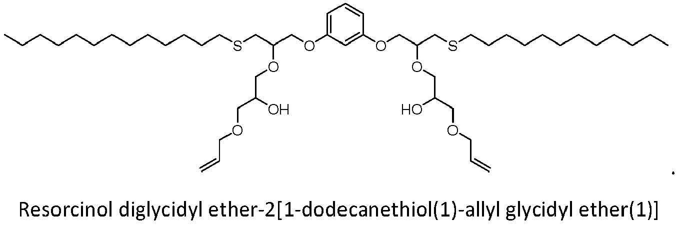

In some aspects, the invention relates to particular reactive surfactant compositions that can be made by the inventive processes. One such composition comprises an unsaturated compound of the formula:

C₃H₅-Ar-O-CH₂CH(R)O-(AO)ₙ-X

wherein Ar is an aryl group, R is a C₈-C₆₄ alkyl or alkenyl group, AO is selected from oxyethylene, oxypropylene, oxybutylenes, and combinations thereof, n has an average value from 1 to 100, and X is selected from hydrogen, alkali metal, ammonium, sulfate, phosphate, and maleate. In more particular aspects, R is C₈-C₂₄ alkyl or C₁₀-C₁₆ alkyl, AO is oxyethylene, n has a value from 2 to 50 or from 2 to 10, and X is hydrogen, sulfate, or phosphate.

As used in this application, "aryl group" refers to an aromatic ring-containing moiety that may be unsubstituted or substituted with one or more alkyl, aryl, aralkyl, alkaryl, halogen, nitro, alkoxy, aryloxy, alkylamino, or haloalkyl groups, or the like.

Compositions of this type are conveniently made by reacting an allyl- or propenyl-substituted phenol with a fatty epoxide followed by alkoxylation and optional capping with a sulfate, phosphate, or maleate group. An exemplary hydrophobe of this type (prior to the alkoxylation step) might have the following structure:

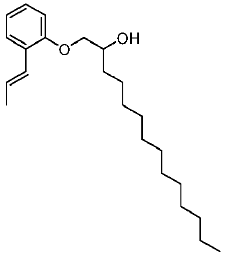

In another particular aspect, the reactive surfactant composition comprises an unsaturated compound of the formula:

C₃H₅-Ar-O-CH₂CH(R)O-CH₂-CH(O-(AO)ₙX)CH₂-OCH₂CH=CH₂

wherein Ar is an aryl group, R is a C₈-C₆₄ alkyl or alkenyl group, AO is selected from oxyethylene, oxypropylene, oxybutylenes, and combinations thereof, n has an average value from 1 to 100, and X is selected from hydrogen, alkali metal, ammonium, sulfate, phosphate, and maleate. In more particular aspects, R is C₈-C₂₄ alkyl or C₁₀-C₁₆ alkyl, AO is oxyethylene, n has a value from 2 to 50 or from 2 to 10, and X is hydrogen, sulfate, or phosphate.

Compositions of this type are conveniently made by reacting an allyl- or propenyl-substituted phenol with a fatty epoxide, followed by reaction of the resulting hydroxy-functional intermediate with allyl glycidyl ether, followed by alkoxylation and optional capping with a sulfate, phosphate, or maleate group. An exemplary hydrophobe of this type (prior to the alkoxylation step) might have the following structure:

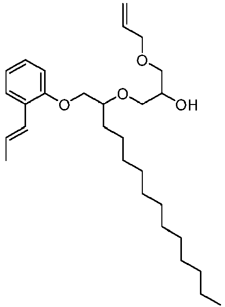

In another particular aspect, the reactive surfactant composition comprises an unsaturated compound of the formula:

R'₂C(Ar(-C₃H₅)-O-CH₂CH(R)O-(AO)ₙ-X)₂

wherein Ar is an aryl group, R is a C₈-C₆₄ alkyl or alkenyl group, each R' is independently hydrogen, C₁-C₆ alkyl, or C₆-C₁₀ aryl, AO is selected from oxyethylene, oxypropylene, oxybutylenes, and combinations thereof, n has an average value from 1 to 100, and X is selected from hydrogen, alkali metal, ammonium, sulfate, phosphate, and maleate. In more particular aspects, R is C₈-C₂₄ alkyl or C₁₀-C₁₆ alkyl, R' is hydrogen, methyl, ethyl, or phenyl, AO is oxyethylene, n has a value from 2 to 50 or from 2 to 10, and X is hydrogen, sulfate, or phosphate.

Compositions of this type are conveniently made by reacting an allyl- or propenyl-substituted bisphenol with two molar equivalents of a fatty epoxide, followed by alkoxylation and optional capping with a sulfate, phosphate, or maleate group. An exemplary hydrophobe of this type (prior to the alkoxylation step) might have the following structure:

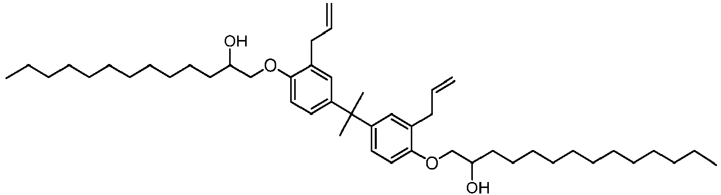

In another particular aspect, the reactive surfactant composition comprises an unsaturated compound of the formula:

C₆H₄(O-CH₂CH(CH₂-O-Ar-C₃H₅)-O-[CH₂CHR-O]ₘ-(AO)ₙ-X)₂

wherein Ar is an aryl group, R is a C₈-C₆₄ alkyl or alkenyl group, AO is selected from oxyethylene, oxypropylene, oxybutylenes, and combinations thereof, n has an average value from 1 to 100, m is 0 or 1, and X is selected from hydrogen, alkali metal, ammonium, sulfate, phosphate, and maleate. In more particular aspects, R is C₈-C₂₄ alkyl or C₁₀-C₁₆ alkyl, AO is oxyethylene, n has a value from 2 to 50 or from 2 to 10, and X is hydrogen, sulfate, or phosphate.

Compositions of this type are conveniently made by reacting a diglycidyl ether such as resorcinol diglycidyl ether with two molar equivalents of an allyl- or propenyl-substituted phenol, followed by reaction with two molar equivalents of a fatty epoxide, followed by alkoxylation and optional capping with a sulfate, phosphate, or maleate group. An exemplary hydrophobe of this type (prior to the alkoxylation step) might have the following structure:

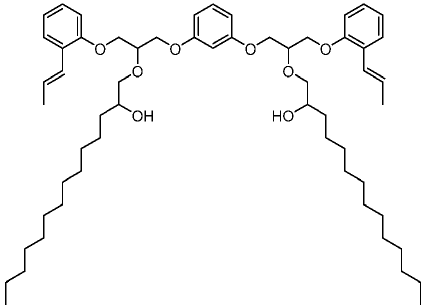

In another particular aspect, the reactive surfactant composition comprises an unsaturated compound of the formula:

M-OOC-CH₂CHR-COO-CH₂-CH₂-Z

or

M-OOC-CH RCH₂-COO-CH₂-CH₂-Z

or

M-OOC-CH=CH-COO-CH₂-CH₂-Z

wherein M is hydrogen, ammonium, an alkali metal, or R²(OCH₂CH₂)ₙ- for which n is 1 to 50 and R² is C₁-C₄ alkyl, particularly methyl, or R² is -COCHRCH₂CO₂CH₂CH₂Z, or R² is -COCH₂CHRCO₂CH₂CH₂Z, or R² is -COCH=CHCO₂CH₂CH₂Z; R is a monounsaturated C₈-C₆₄ group; and Z is an N-imidazolidinone group. In some aspects, M is ammonium and R is C₈-C₂₄ alkyl or a C₁₀-C₁₆ group. Exemplary compositions of this type could have the following structures: 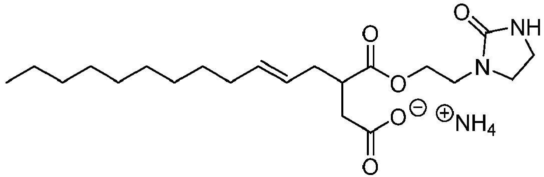 or 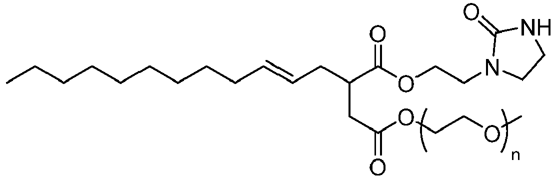 or 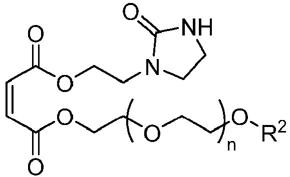 or 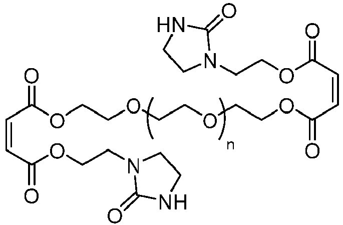 or 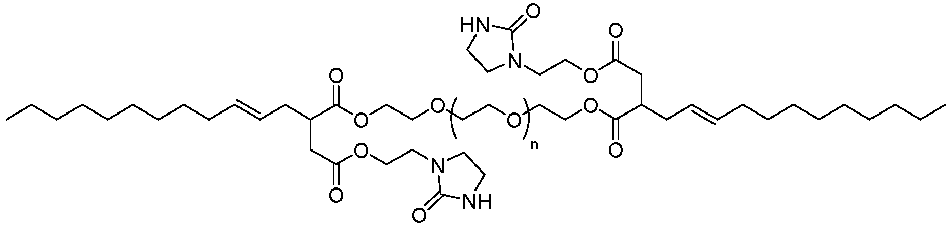 in which n has a value within the range of 1 to 50. See Examples 38 and 39, below, for ways to synthesize some of these compositions.

In another aspect, an alkoxylate prepared as described in this application is further reacted with a succinic acid 1-(2-hydroxyethyl)imidazolidinone monoester. The same kind of product can be made by reacting the alkoxylate with succinic anhydride followed by coupling to 2-hydroxyethylethyeneurea in the presence of a dehydrating agent such as N,N-dicyclohexylcarbodiimide. For example, reaction of 2-allylphenol with 1,2-epoxytetradecane followed by reaction with one equivalent of EO gives an ethoxylate that can be esterified with succinic anhydride followed by coupling to 2-hydroxyethylethyeneurea to give a reactive surfactant with the following structure:

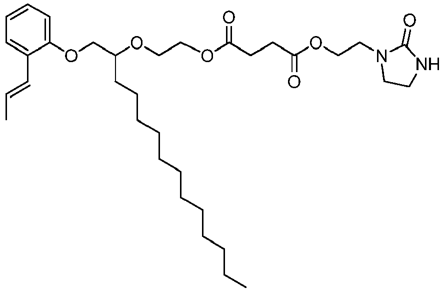

The hydrophilicity of similar compositions can be adjusted by alkoxylating with any desired number of molar equivalents of EO or any combination of EO and propylene oxide (PO). See Examples 1 and 37 below.

The invention includes another process for making a reactive surfactant. This process comprises reacting an alkyl- or alkenylsuccinic anhydride with an olefin-functional nucleophile as previously described to produce an olefin-functional succinate monoester. Thus, in some aspects, the olefin-functional nucleophile is an olefin-functional phenol, an olefin-functional polyphenol, an olefin-functional alcohol, an olefin-functional polyalcohol, alkoxylates thereof, or mixtures thereof as previously described. In other aspects, the olefin-functional nucleophile is a hydroxy-functional acrylate, a hydroxy-functional acrylamide, an alkoxylate thereof, or a mixture thereof, as previously described.

Optionally, the monoester is then neutralized with a base (e.g., NaOH, KOH, ammonia) to give the corresponding monoester/acid salt. In the scheme below, "NuH" is an olefin-functional nucleophile, R represents a residue from the nucleophile minus a hydrogen atom, and ammonia is the neutralizing agent:

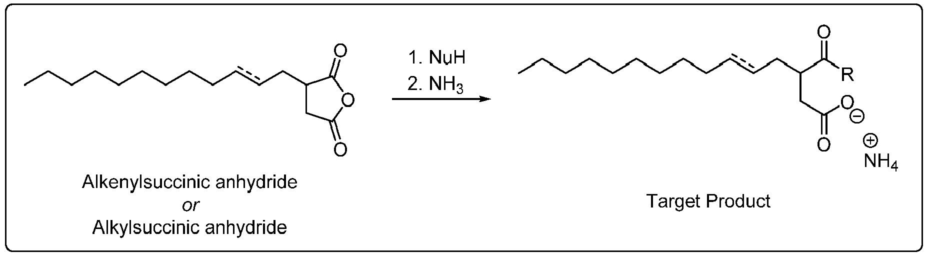

Methods of making the alkyl- or alkenylsuccinic anhydrides are well known. In one suitable process, maleic anhydride and an alpha-olefin are heated under conditions effective to promote an ene reaction and produce an alkenylsuccinic anhydride.

The reaction of an alkenylsuccinic anhydride with 2-hydroxyethyl methacrylate ("HEMA") followed by neutralization with ammonia is illustrative:

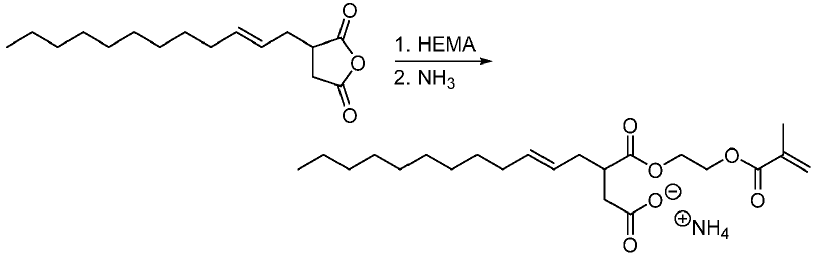

Similarly, an olefin-functional hydrophobe can be produced by reacting an alkenylsuccinic anhydride with ethylene glycol monoallyl ether followed by neutralization:

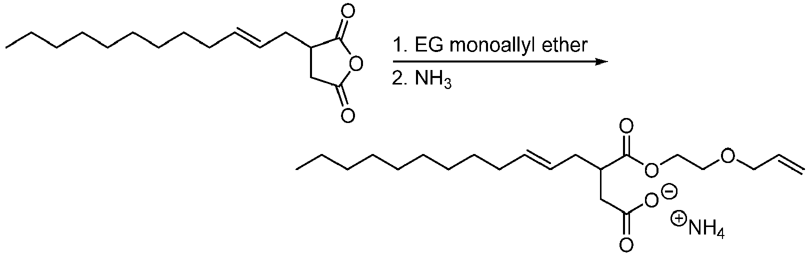

In other aspects, the invention relates to polymerizable mixtures comprising any of the above reactive surfactant compositions and an acrylic monomer. Suitable acrylic monomers are well known and include, for example, acrylic acid, methacrylic acid, methyl acrylate, methyl methacrylate, ethyl acrylate, ethyl methacrylate, butyl acrylate, butyl methacrylate, isobutyl acrylate, isobutyl methacrylate, hexyl acrylates, hexyl methacrylates, 2-ethylhexyl acrylate, 2-ethylhexyl methacrylate, and the like, and mixtures thereof. A combination of butyl acrylate, methyl methacrylate, and acrylic acid is particularly useful.

Polymerization of the acrylic monomer(s) and the reactive surfactant according to well-known methods for emulsion polymerization provides an aqueous latex emulsion of the invention. Suitable methods for performing the emulsion polymerization appear in the examples below. The latex emulsions are particularly valuable for making paints and coatings having superior physical and/or mechanical properties. As shown in the examples below, the inventive reactive surfactants deliver stable latex emulsions with reduced tendency for surfactant migration or excessive foaming. Coatings from the emulsions have improved wet adhesion, scrub resistance, and water resistance.

The following examples merely illustrate the invention; the skilled person will recognize many variations that are within the spirit of the invention and scope of the claims.

### EXAMPLE 1: Reactive Surfactant from 2-Allylphenol, 1,2-Epoxytetradecane, and Ethylene Oxide

A 5-L flask equipped with agitator, condenser, thermocouple, heating mantle, nitrogen inlet, and Dean-Stark trap is charged with 2-allylphenol (1042 g, 7.8 mol) and sparged with nitrogen for 10 min. Potassium methoxide (55 g of 25% solution in methanol) is added. The reactor contents are heated to 135 °C under a flow of nitrogen over 2 h with removal of methanol. The Dean-Stark trap is replaced by an addition funnel containing VIKOLOX^{®} 14 (1,2-epoxytetradecane, 1654 g, 7.8 mol, product of Arkema). The mixture is heated to 145 °C, and the fatty epoxide is added slowly over 1.5 h while maintaining the reaction temperature within the range of 142-148 °C. The mixture is held for an additional 3 h at 145 °C, after which ¹H NMR analysis shows complete conversion of the fatty epoxide.

The fatty alcohol reaction product (2424 g, 7.0 mol) is transferred to a 2-gal pressure reactor equipped with agitator, nitrogen inlet, and hot-oil jacket. The reactor contents are purged with nitrogen and heated to 140-160 °C while maintaining a reactor pressure within the range of 30-80 psig. Ethylene oxide (1540 g, 35 mol) is charged slowly to the reactor over 3 h. The reactor is held at 140 °C for 2 h to ensure complete reaction of the EO. The reactor is sampled to remove a 5 mole EO/mole fatty alcohol ethoxylate product. Additional fatty alcohol ethoxylates having ethoxylation levels of 10, 15, 20, or 25 moles EO per mole of fatty alcohol are produced in cycles of ethoxylation followed by a 2 h cook-down as previously described. After the 25 mole EO/mole fatty alcohol product is made, the reactor is cooled to 25 °C. ¹H NMR analysis shows successful incorporation of EO and quantitative isomerization of the allyl group to a propenyl group in the 5 moles EO/mole product and each subsequent product.

### EXAMPLE 2: Reactive Ether Sulfate Surfactant

A sample of 10 mole ethoxylate from Example 1 (184 g, 233 mmol) is charged to a round-bottom flask equipped with agitator, thermocouple, heating mantle, and nitrogen inlet. The ethoxylate is heated to 60 °C, and sulfamic acid (22.6 g, 233 mmol) is added with stirring under a nitrogen sparge. The reaction temperature is slowly increased to 105 °C and held for 4 h. Analysis by ¹H NMR and acid-base titration shows that the reaction is complete. Diethanolamine (1.7 g) is added to neutralize the mixture to pH 7 (as measured in 10% aq. isopropanol). The ether sulfate reaction product (180 g) is dissolved in warm deionized water (700 g), and preservative (1.1 g of NEOLONE^{™} M-10, product of Dow) is added. Vacuum oven solids content (50 °C, 2h): 21.3%. As-is pH: 6.6.

### EXAMPLE 3: Reactive Surfactant from Tristyrylphenol, Phenyl Glycidyl Ether, Allyl Glycidyl Ether, and Alkylene Oxides (EO/PO)

Tristyrylphenol (1345 g, 3.5 mol, product of Levaco Chemicals), which contains about 75% tristyrylphenol/25% distyrylphenol, is charged to a 6-L flask equipped with agitator, condenser, thermocouple, heating mantle, nitrogen inlet, and Dean-Stark trap. The reactor contents are heated to 65 °C, and the headspace is sparged with nitrogen for 10 min. Potassium methoxide (50 g of 25% solution in methanol) is added. The reactor contents are heated to 135 °C under a flow of nitrogen over 2 h with removal of methanol. The Dean-Stark trap is replaced by an addition funnel containing phenyl glycidyl ether ("PGE," 523 g, 3.5 mol, product of TCI Chemicals). The mixture is heated to 145 °C, and PGE is added slowly over 75 min. while maintaining the reaction temperature within the range of 145-153 °C. The mixture is held for an additional 1.5 h at 145 °C, after which ¹H NMR analysis shows complete conversion of the PGE. The reaction mixture is allowed to cool to 125 °C. Allyl glycidyl ether ("AGE," 792 g, 6.9 mol) is then added from the addition funnel over 50 min. while keeping the reaction mixture at 125-130 °C. After 3 h, only traces of the AGE remain. The mixture is then cooled to room temperature.

A sample of the TSP-PGE-AGE adduct (2302 g, 3.0 mol) is transferred to the 2-gal pressure reactor described in Example 1, and the same general procedure is used for alkoxylation, in this case, using a 2:1 molar mixture of ethylene oxide (EO) and propylene oxide (PO). The epoxide mixture is added slowly to the reactor at 120-145 °C while maintaining a reactor pressure from 30-80 psig. Various levels of alkoxylation are used to provide 10, 15, 20, and 25 mole alkoxylates/mole of TSP-PGE-AGE adduct. As in Example 1, a 2-h cook-down period follows each alkylene oxide ("AO") addition. To produce the 10 mole AO/mole TSP-PGE-AGE product, 1450 g of mixed EO/PO feed is used. Alkoxylated products having Mₙ values (by gel permeation chromatography) of 1000, 1200, 1400, and 1600 g/mol and corresponding M_{w}/Mₙ values of 1.18, 1.21, 1.24, and 1.26 are produced.

### EXAMPLE 4: Reactive Ether Sulfate Surfactant

The procedure of Example 2 is generally followed using a sample of 10 mole alkoxylate from Example 3 (155.5 g, 144 mmol) and sulfamic acid (14.0 g, 144 mmol). The reaction temperature is slowly increased to 95 °C and held for 8 h. Analysis by acid-base titration shows that the reaction is complete. Diethanolamine (0.7 g) is added to neutralize the mixture to pH 7. The ether sulfate reaction product (171.4 g) is dissolved in warm deionized water (630 g), and preservative (0.9 g of NEOLONE^{™} M-10) is added. Vacuum oven solids content (50 °C, 2h): 21.3%. As-is pH: 6.6.

### COMPARATIVE EXAMPLE 5L:

### Latex Prepared using Conventional Surfactant

This example illustrates the preparation of a conventional latex using 2.0 wt.% of sodium nonylphenol 10 EO ethoxylate sulfate as the anionic surfactant.

A round-bottom flask equipped with agitator, heating mantle, thermocouple, temperature controller, and nitrogen inlet is charged with deionized water (296 g) and STEOL^{®} EP-110K (sodium nonylphenol 10 EO sulfate, 32.8% solids, product of Stepan Company, 6.9 g). The contents are heated to 83 °C.

Separately, a monomer emulsion ("ME") is prepared by adding with vigorous agitation a mixture of butyl acrylate (217 g), methyl methacrylate (278 g), and methacrylic acid (5 g) to a mixture of STEOL^{®} EP-110K (23.6 g) in deionized water (137 g) and stirring for 10 min. to form a stable emulsion.

An *in-situ* latex seed is prepared by adding a portion of the ME (33 g) to the reaction flask, followed by a solution of ammonium persulfate (1.0 g) and sodium bicarbonate (0.5 g) in deionized water (20 g). Within 3 min. after adding the persulfate initiator solution, the mixture exotherms to about 85 °C, indicating polymerization of the monomers. After 10 min., a sample of the reaction mixture is analyzed by dynamic light scattering, which shows an average particle size distribution for the latex seed of 45 nm.

After 12 min., the ME is added using a metering pump over 3 h concurrently with addition of a solution of ammonium persulfate (2.7 g) and sodium bicarbonate (1.5 g) in deionized water (75 g). The reaction temperature is maintained at 83 °C. After 3 h, the ME and initiator feed additions are complete. The ME addition line is flushed with water (50 g) into the reactor. The reaction is held at 83 °C for an additional hour, then cooled to room temperature. The pH of the resulting latex is adjusted from 5.4 to 7.5 with dilute ammonium hydroxide followed by the addition of NEOLONE^{™} M-10 preservative (1.2 g). The latex is filtered through a 100-mesh screen, and coagulum (0.05 g) is collected. The reactor is free of coagulum build-up. Average latex particle size: 119 nm.

### EXAMPLE 6L:

### Latex from the Reactive Surfactant of Ex. 2

The procedure of Comparative Example 5L is generally followed. The reactive surfactant prepared in Example 2 (13.0 g) and deionized water (291 g) are added to the reaction flask. The ME is prepared by adding with vigorous agitation the mixture of butyl acrylate, methyl methacrylate, and methacrylic acid described earlier to a mixture of reactive surfactant (33.5 g) and deionized water (127 g), followed by stirring for 10 min. to form a stable emulsion.

An *in-situ* latex seed is prepared as described above by adding a portion of the ME (33 g) to the reaction flask, followed by the solution of ammonium persulfate and sodium bicarbonate in deionized water. The mixture exotherms to about 85 °C, and after 10 min., an in-situ seed having an average particle size distribution 42 nm forms.

The ME and initiator solutions are added at 83°C, followed by a 1-hour cook, as previously described. The pH of the resulting latex is adjusted from 5.3 to 7.4 with dilute ammonium hydroxide followed by the addition of NEOLONE^{™} M-10 preservative (1.2 g). The latex is filtered through a 100-mesh screen, and coagulum (0.08 g) is collected. The reactor is free of coagulum build-up. Average latex particle size: 101 nm.

### COMPARATIVE EXAMPLE 7L:

### Latex from a Comparative Reactive Surfactant

The procedure of Comparative Example 5L is generally followed except that HITENOL^{®} BC-1025 surfactant (4-nonylphenol-2-(1-propen-1-yl) polyoxyethylene 10 EO ether sulfate, ammonium salt, 25% solids, product of Dai-ichi Kogyo Seiyaku, 9.5 g) is used instead of STEOL^{®} EP-110K.

The ME is prepared by adding with vigorous agitation the mixture of butyl acrylate, methyl methacrylate, and methacrylic acid described earlier to a mixture of HITENOL^{®} BC-1025 surfactant (30.5 g) and deionized water (130 g), followed by stirring for 10 min. to form a stable emulsion.

An *in-situ* latex seed is prepared as described above by adding a portion of the ME (33 g) to the reaction flask, followed by the solution of ammonium persulfate and sodium bicarbonate in deionized water. The mixture exotherms to about 85 °C, and after 10 min., an in-situ seed having an average particle size distribution 42 nm forms.

The ME and initiator solutions are added at 83 °C, followed by a 1-hour cook, as previously described. The pH of the resulting latex is adjusted from 5.3 to 7.3 with dilute ammonium hydroxide followed by the addition of NEOLONE^{™} M-10 preservative (1.2 g). The latex is filtered through a 100-mesh screen, and coagulum (0.02 g) is collected. The reactor is free of coagulum build-up. Average latex particle size: 109 nm. Latex solids content: 45.5 wt.%.

### Water Resistance of Latex Coatings

The latexes of Comparative Example 5L, Example 6L, and Comparative Example 7L, each having a pH of 7.2, are drawn down as clear, wet films on black Leneta panels using a #22 wire wound rod. The films are dried at room temperature for 1 h, cured in a 60 °C oven for 15 min., then stored at room temperature for 15 min. The coated panels are then submerged in 60 °C deionized water for 1 h, followed by cooling to room temperature for 2 h. The film from the latex of Comparative Example 5L (conventional surfactant) becomes milky white, indicating severe water sensitivity.

The coated panels are stored for 3 weeks in water. The coating from the latex of Example 6L is essentially blister-free, while the coatings from the latexes of Comparative Examples 5L (conventional surfactant) and 7L (commercial reactive surfactant) show significant blistering. The results show that the inventive reactive surfactant enables the production of latex coatings with improved adhesion and water resistance.

### Foaming Properties

A sample (10 g) of each of the latexes of Comparative Example 5L, Example 6L, and Comparative Example 7L is added to a 20-mL scintillation vial and is shaken by hand for 1 min. The latex of Example 6L exhibits lower foaming that that of Comparative Example 7L, which exhibits lower foaming than that of Comparative Example 5L. Low foaming is desirable because it improves latex processing and performance in paint and coating applications. Low foaming suggests incorporation of a surfactant into the latex polymer.

### EXAMPLE 8:

### Reactive Phosphate Ester Surfactant

A sample of the 5 EO fatty alcohol ethoxylate from Example 1 (114.3 g, 202 mmol) is charged to a round-bottom flask equipped with agitator, thermocouple, heating mantle, and nitrogen inlet. The reactor contents are warmed to 42 °C, and phosphorus pentoxide (9.6 g, 68 mmol) is added over 10 min. with stirring. The reaction mixture exotherms to 50°C. The mixture is heated to 80 °C and is held for 1 h, followed by heating to 95 °C for 3 h. ³¹P NMR analysis shows that the reaction is complete. The reactive phosphate ester surfactant is used to produce the latex of Example 15L, below.

### COMPARATIVE EXAMPLE 9:

### Attempted Synthesis of Ethoxylate from 2-Allylphenol/Fatty Glycidyl Ether Adduct

The procedure of Example 1 of U.S. Pat. No. 9,376,510 is initially followed in an attempt to make a glycidyl ether by reacting 2-allylphenol with epichlorohydrin catalyzed by boron trifluoride diethyl etherate. However, when the 2-allylphenol is heated in the presence of the BF₃ diethyl etherate, an unexpected, out-of-control exothermic reaction occurs (before any epichlorohydrin can be added). The reaction is thereafter considered too risky to pursue, and alternative synthetic routes are considered.

The target molecule is next sought from an initial reaction of 2-allylphenol with a fatty glycidyl ether, followed by ethoxylation, as follows.

2-Allylphenol (1079 g, 7.85 mol) is charged to a round-bottom flask equipped with agitator, condenser, heating mantle, thermocouple, nitrogen inlet, and Dean-Stark trap. The reactor is sparged with nitrogen for 10 min. Potassium methoxide (56 g of 25% KOMe in methanol) is added. The reaction mixture is heated to 135 °C with nitrogen sparging to remove methanol over 2 h. The Dean-Stark trap is then replaced by an addition funnel containing the glycidyl ether of a C₁₂-C₁₄ alcohol (Aldrich, 1725 g). The reaction temperature is increased to 145 °C, and the fatty glycidyl ether is slowly added over 2 h while maintaining the reaction temperature within the range of 145-150 °C. When the glycidyl ether addition is complete, the mixture is held at 150 °C for 3 h. ¹H NMR analysis shows about 75% conversion, which is not improved by further heating. Flake KOH (6.0 g) is added to the reactor, and after another 1.5 h, epoxide conversion reaches 95%.

A portion of the reaction product (2500 g) is transferred to the 2-gallon pressure reactor described previously. The reactor contents are purged with nitrogen and heated to 140-160 °C while maintaining a reactor pressure within the range of 30-80 psig. Ethylene oxide (10 g) is charged to the reactor, and the pressure increases from 30 to 40 psig. After 1 h, there has been no pressure drop, indicating that the EO has not reacted. Several additional attempts are also unsuccessful.

### EXAMPLE 10:

### Preparation of a Wet-Adhesion Promoting Reactive Surfactant

A 1-L round-bottom flask equipped with agitator, heating mantle, thermocouple, and heat controller is charged with toluene (106 g) and 1-(2-hydroxyethyl)-2-imidazolidinone (as supplied, 75% aq. solution from Aldrich, stripped to 300 ppm moisture content, 55.8 g, 429 mmol). The solution is heated to 58 °C, and triethylamine (43.5 g, 431 mol, 300 ppm moisture content) is added to the flask with stirring. 2-Dodecyl-1-yl succinic anhydride (TCI Japan, 114 g, 429 mmol) in toluene (100 g) is slowly added over 1 h while maintaining the reaction temperature at about 50 °C. The mixture is heated to 70 °C for 1.5 h, after which FTIR analysis shows disappearance of the absorption band for the anhydride carbonyl stretch at 1785 cm⁻¹.

A portion of the reaction mixture (210.4 g) is transferred to a flask equipped for vacuum distillation. The reactor is heated to 40-50 °C, and a vacuum of 2.5 Torr (2.5 mm Hg) is applied for 1 h to remove toluene and triethylamine. The reaction product is a light-yellow viscous liquid at 40 °C. The product is stored at 70 °C overnight, then slowly added to deionized water (260 g) containing concentrated ammonium hydroxide (6.2 g) to adjust the pH from 5.8 to 7.0. Solids content (vacuum, 2 h): 24.3%.

### EXAMPLE 11L:

### Latex Synthesis from Reactive Surfactant

The procedure of Comparative Example 5L is generally followed. The reactive surfactant prepared in Example 10 (4.4 g) and deionized water (298 g) are added to the reaction flask. The ME is prepared by adding with vigorous agitation the mixture of butyl acrylate, methyl methacrylate, and methacrylic acid described earlier to a mixture of reactive surfactant (46.2 g) and deionized water (118 g), followed by stirring for 10 min. to form a stable emulsion.

An *in-situ* latex seed is prepared as described above by adding a portion of the ME (33 g) to the reaction flask, followed by the solution of ammonium persulfate and sodium bicarbonate in deionized water. The mixture exotherms to about 86 °C, and after 10 min., an in-situ seed having an average particle size distribution 54 nm forms.

The ME and initiator solutions are added at 83 °C, followed by a 1-hour cook, as previously described. The pH of the resulting latex is adjusted from 5.7 to 7.3 with dilute ammonium hydroxide followed by the addition of NEOLONE^{™} M-10 preservative (1.2 g). The latex is filtered through a 100-mesh screen, and coagulum (0.08 g) is collected. The reactor is free of coagulum build-up. Average latex particle size: 109 nm. Solids content: 45.4 wt.%.

### COMPARATIVE EXAMPLE 12L

### Latex Synthesis using Wet-Adhesion Monomer with Conventional Surfactant

The procedure of Comparative Example 5L is generally followed using a conventional surfactant (POLYSTEP^{®} A-15) and a wet-adhesion monomer (SIPOMER^{®} WAM II) as follows.

POLYSTEP^{®} A-15 (sodium dodecylbenzene sulfonate, 22.9% solids, product of Stepan Company, 5.0 g) and deionized water (300 g) are added to the reaction flask.

Separately, the ME is prepared by adding with vigorous agitation a mixture of butyl acrylate (260 g), methyl methacrylate (230 g), and acrylic acid (10 g) to a mixture of POLYSTEP^{®} A-15 (16.8 g) and SIPOMER^{®} WAM II (methacrylamido ethylimidazolidinone, 46%; methacrylic acid, 25%; and water, 29%; product of Solvay, 5.0 g) in deionized water (136 g) and stirring for 10 min. to form a stable emulsion.

An *in-situ* latex seed is prepared as described above by adding a portion of the ME (33 g) to the reaction flask, followed by the solution of ammonium persulfate and sodium bicarbonate in deionized water. The mixture exotherms to about 86 °C, and after 10 min., an in-situ seed forms.

The ME and initiator solutions are added at 83 °C, followed by a 1-hour cook, as previously described. The pH of the resulting latex is adjusted from 3.9 to 7.5 with dilute ammonium hydroxide followed by the addition of NEOLONE^{™} M-10 preservative (1.2 g). The latex is filtered through a 100-mesh screen, and coagulum (0.05 g) is collected. The reactor is free of coagulum build-up. Average latex particle size: 123 nm. Solids content: 45.3 wt.%.

### COMPARATIVE EXAMPLE 13L

### Latex Synthesis using Conventional Surfactant; No Wet-Adhesion Component

The procedure of Comparative Example 12L is generally followed except that the SIPOMER^{®} WAM II monomer mixture is omitted. Particle size: 119 nm. Solids content: 46.2 wt.%.

### EXAMPLE 14

### Latex Paint Formulations and Evaluation

### Preparation and application of paints

Latex paints are formulated from a master batch prepared by combining, with mechanical stirring, deionized water (163 g) and a pre-dispersed slurry of titanium dioxide (TI-PURE^{™} R-746, 76.6% solids, product of DuPont, 526 g). POLYSTEP^{®} TD-129 wetting agent (product of Stepan, 2.5 g), propylene glycol (9.2 g), and BYK-024 defoamer (product of BYK Additives, 3.6 g) are added. After mixing for 15 min., equal amounts of the pre-mix (210 g) are poured into three smaller jars. To each jar is added with stirring one of the latexes of Example 11L or Comparative Examples 12L and 13L (103 g on a 100% solids basis). Coalescing solvent (TEXANOL^{™} ester alcohol; 2,2,4-trimethyl-1,3-pentanediol monoisobutyrate, product of Eastman, 5.0 g) is then added to each paint formulation over 2 min., followed by 30 min. of mixing. Ammonium hydroxide added to increase the pH to 9.0. The paints are thickened to viscosities of 85 to 90 KU units with a combination of ACROYSOL^{™} RM-8W and RM 2020NPR (products of Dow) HEUR-type rheology modifiers at about 0.4 and 1.1 wt.%, respectively, on a 100% solids basis for the total paint composition. After 20 min. of mixing, NEOLONE^{™} M-10 preservative (0.5 g) added. Solids content: 48 wt.%.

Formulated paints are applied, using a 7-mil Dow wet-film applicator, to black Leneta panels previously coated with a tinted all-surface, oil-based enamel (Sherwin-Williams). The enamel is coated onto the Leneta panels using a 7-mil Dow wet-film applicator and aged for 1 month prior to use. The formulated paint coatings are aged for one week before testing.

### Wet-scrub resistance

The dry, coated panels are cut with a straight-edge blade into 2"-wide panels. The panels are positioned on a Gardner straight-line washability machine and are subjected to 1000 scrub cycles according to modified ASTM 2486 using water and the abrasive scrub compound specified in the method. Coatings from the latexes of Example 11L (inventive reactive surfactant used) and Comparative Example 12L (wet adhesion monomer included with conventional surfactant) are intact after 1000 cycles. The coating from the latex of Comparative Example 13L (conventional surfactant, no wet adhesion monomer included) fails within 200 cycles.

### EXAMPLE 15L

### Latex Synthesis Using Reactive Phosphate Ester Surfactant

The procedure of Comparative Example 5L is generally followed using the reactive phosphate ester surfactant prepared in Example 8, as follows.

The reactive phosphate ester (2.0 g) and deionized water (301 g) are added to the reaction flask, and the mixture is neutralized to pH 7.2 with dilute aq. ammonium hydroxide (1.2 g).

Separately, the ME is prepared by adding with vigorous agitation a mixture of butyl acrylate (260 g), methyl methacrylate (230 g), and acrylic acid (10 g) to a mixture of the reactive phosphate ester surfactant (13.1) and deionized water (153 g) and stirring for 10 min. to form a stable emulsion.

An *in-situ* latex seed is prepared as described above by adding a portion of the ME (33 g) to the reaction flask, followed by the solution of ammonium persulfate and sodium bicarbonate in deionized water. The mixture exotherms to about 86 °C, and after 10 min., an in-situ seed with a particle size of 48 nm forms.

The ME and initiator solutions are added at 83 °C, followed by a 1-hour cook, as previously described. The pH of the resulting latex is adjusted from 5.3 to 7.5 with dilute ammonium hydroxide followed by the addition of NEOLONE^{™} M-10 preservative (1.2 g). The latex is filtered through a 100-mesh screen, and coagulum (0.06 g) is collected. The reactor is free of coagulum build-up. Average latex particle size: 116 nm. Solids content: 45.0 wt.%.

### COMPARATIVE EXAMPLE 16L

### Latex Synthesis Using a Conventional Phosphate Ester Surfactant

The procedure of Example 15L is generally followed, except that 1.5 wt.% of POLYSTEP^{®} P-12A (tridecyl alcohol 6 EO phosphate ester, ammonium salt, product of Stepan) is used instead of the inventive reactive phosphate ester surfactant.

### Water resistance of latex coatings

The latexes of Example 15L and Comparative Example 16L are drawn down on black Leneta panels as previously described to produce wet films. The films are dried at room temperature for 1 h, cured in a 60 °C oven for 15 min., then stored at room temperature for 15 min. The coated panels are then submerged in 60 °C deionized water for 1 h, followed by cooling to room temperature for 2 h. The film from the latex of Example 15L (reactive phosphate ester surfactant) develops less opacity when compared with the control sample, indicating improved water resistance from the latex made with the reactive surfactant.

### Contact angle

The latexes of Example 15L and Comparative Example 16L are drawn down on Mylar panels using a #22 wire wound rod. The films are dried overnight under ambient conditions. Contact angles are measured using a Kruss Mobile Surface Analyzer. The analyzer is adjusted to deliver one microliter of purified water per droplet. Droplets are applied to the films and the contact angle measurement is made after 15 seconds. Ten measurements are made for each film, and the results are averaged.

The film from the latex of Example 15L exhibits a contact angle of 75 degrees, while the latex of Comparative Example 16L exhibits a contact angle of 37 degrees. The higher contact angle from the latex of Example 15L indicates a more hydrophobic coating. Covalent bonding of the reactive surfactant could account for reduced migration of the surfactant and fewer hydrophilic domains that would be susceptible to water penetration.

### EXAMPLE 17:

### Hydrophobe from 1,2-Epoxytetradecane and 1,4-Butanediol Vinyl Ether

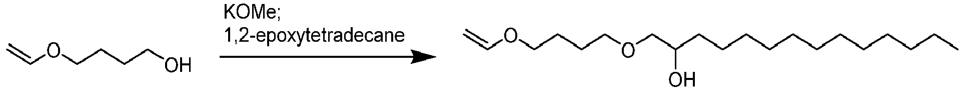

A 500-mL flask is placed in a heating mantle and is fitted with an overhead stirrer, a thermocouple with attached temperature controller, a nitrogen inlet/sparging tube, and an addition funnel fitted with a gas outlet plumbed to an oil bubbler. The addition funnel is charged with 1,2-epoxytetradecane (161.6 g, 761 mmol). Under a flow of nitrogen, the flask is charged with solid potassium methoxide (2.03 g, 28.9 mmol) and 1,4-butanediol vinyl ether (88.4 g, 761 mmol). The resulting mixture is heated to 110 °C, at which point 1,2-epoxytetradecane is added from the addition funnel over 17 min. After stirring for 3 h and 40 min., an aliquot is removed and analyzed. ¹H NMR shows 66% consumption of the epoxide. The reaction temperature is increased to 120 °C. After stirring at that temperature for 19 h, ¹H NMR analysis shows complete consumption of the epoxide. The hot reaction mixture is poured into a jar and allowed to cool to room temperature to give the desired secondary fatty alcohol hydrophobe (244 g, 97.7%). The product contains 0.45% K+ by mass.

### EXAMPLE 18

### Hydrophobe from Cinnamyl Alcohol and 1,2-Epoxytetradecane

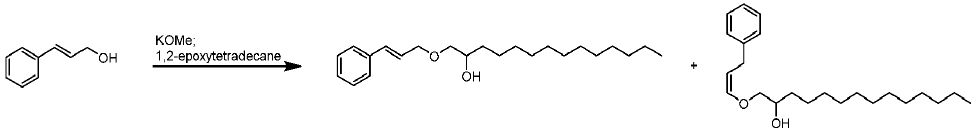

A 500-mL flask is equipped as in Example 17. The addition funnel is charged with 1,2-epoxytetradecane (153.9 g, 725 mmol). Under a flow of nitrogen, the flask is charged with solid potassium methoxide (2.04 g, 29.1 mmol) and trans-cinnamyl alcohol (97.2 g, 724 mmol). The resulting mixture is heated to 110 °C, at which point addition of 1,2-epoxytetradecane begins. As the addition proceeds, the reaction temperature is slowly increased, reaching 140 °C after 33 min. Addition of 1,2-epoxytetradecane continues for another 23 min. After stirring at that temperature for 17.5 h, ¹H NMR shows complete consumption of the epoxide. The hot reaction mixture is poured into a jar and allowed to cool to room temperature to give a 1:1 mixture of the isomeric products: *trans*-cinnamyl alcohol-[1,2-epoxytetradecane(1)] and *cis*-3-phenylprop-1-en-1-ol-[1,2-epoxytetradecane(1)] (231 g, 92.0%). The product contains 0.45% K+ by mass.

### EXAMPLE 19

### Hydrophobe from Eugenol and 1,2-Epoxytetradecane

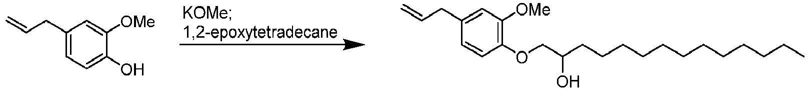

A 500-mL flask is equipped as in Example 17. The addition funnel is charged with 1,2-epoxytetradecane (119.2 g, 561 mmol). Under a flow of nitrogen, the flask is charged with solid potassium methoxide (1.62 g, 23.1 mmol) and eugenol (92.2 g, 561 mmol). The resulting mixture is heated to 120 °C, at which point 1,2-epoxytetradecane is added from the addition funnel over 29 min. After stirring for 18 h, ¹H NMR shows complete consumption of the epoxide. The hot reaction mixture is poured into a jar and allowed to cool to room temperature to give the desired secondary fatty alcohol hydrophobe (205 g, 96.8%). The product contains 0.43% K+ by mass.

### EXAMPLE 20

### Hydrophobe from Resorcinol Diglycidyl Ether and 2-Allylphenol

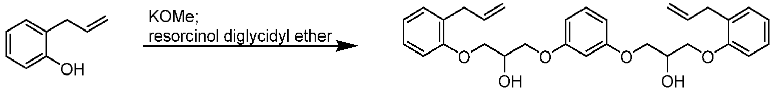

A 500-mL flask is equipped as in Example 17 except that an addition funnel is not included. Under a flow of nitrogen, the flask is charged with solid potassium methoxide (1.62 g, 23.1 mmol) and 2-allylphenol (109.6 g, 816 mmol). The resulting mixture is heated to 110 °C, at which point resorcinol diglycidyl ether (90.1 g, 405 mmol) is added in portions via pipette over 18 min. During the addition, the reaction temperature is maintained at or below 123 °C by raising and lowering the heating mantle. After stirring at 123 °C for 18 h, ¹H NMR shows complete consumption of the epoxide. The hot reaction mixture is poured into a jar and allowed to cool to room temperature to give the desired diol (193 g, 97.1%). The product contains 0.45% K+ by mass.

### EXAMPLE 21

### Hydrophobe from Trimethylolpropane Allyl Ether and 1,2-Epoxytetradecane

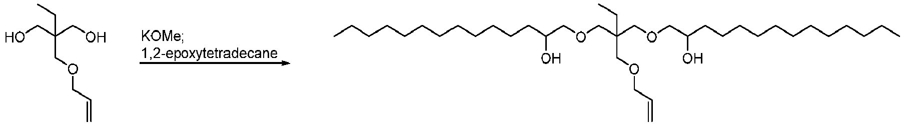

A 500-mL flask is equipped as in Example 17. The addition funnel is charged with 1,2-epoxytetradecane (142 g, 669 mmol). Under a flow of nitrogen, the flask is charged with solid potassium methoxide (1.63 g, 23.2 mmol) and trimethylolpropane allyl ether (58.3 g, 334 mmol). The resulting mixture is heated to 120 °C, at which point 1,2-epoxytetradecane is added from the addition funnel over 24 min. After stirring for 20.5 h at that temperature, ¹H NMR shows complete consumption of the epoxide. The hot reaction mixture is poured into a jar and allowed to cool to room temperature to give the desired fatty diol hydrophobe (197 g, 98.2%). The product contains 0.45% K+ by mass.

### EXAMPLE 22

### Hydrophobe from 2,2'-Diallylbisphenol A and 1,2-Epoxytetradecane

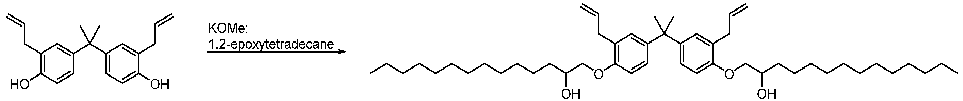

A 500-mL flask is equipped as in Example 17. The addition funnel is charged with 1,2-epoxytetradecane (117 g, 655 mmol). Under a flow of nitrogen, the flask is charged with solid potassium methoxide (1.62 g, 23.1 mmol) and 2,2'-diallylbisphenol A (84.9 g, 275 mmol). The resulting mixture is heated to 120 °C, at which point 1,2-epoxytetradecane is added from the addition funnel over 27 min. After stirring for 23 h at that temperature, ¹H NMR shows complete consumption of the epoxide. The hot reaction mixture is poured into a jar and allowed to cool to room temperature to give the desired fatty diol hydrophobe (196 g, 97.3%). The product contains 0.44% K+ by mass.

### EXAMPLE 23

### Hydrophobe from Pterostilbene and 1,2-Epoxytetradecane

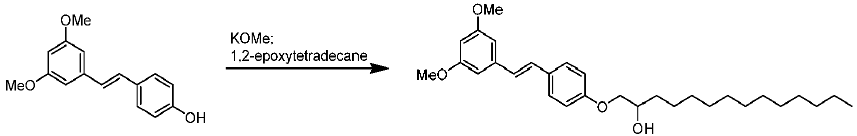

A 500-mL flask is equipped as in Example 17. The addition funnel is charged with 1,2-epoxytetradecane (82.1 g, 387 mmol). Under a flow of nitrogen, the flask is charged with solid potassium methoxide (1.47 g, 21 mmol) and pterostilbene (98.9 g, 386 mmol). The resulting mixture is heated to 110 °C, at which point 1,2-epoxytetradecane is added from the addition funnel over 27 min. After stirring for 22.5 h at that temperature, ¹H NMR shows complete consumption of the epoxide. The hot reaction mixture is poured into a jar and allowed to cool to room temperature to give the desired fatty hydrophobe (178 g, 98.7%). The product contains 0.45% K+ by mass.

### EXAMPLE 24

### Hydrophobe from Eugenol and Tristyrylphenol Glycidyl Ether

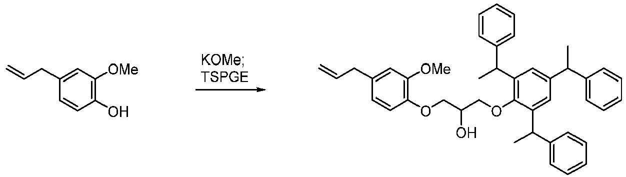

A 500-mL flask is equipped as in Example 17 except that an addition funnel is not included. Under a flow of nitrogen, the flask is charged with solid potassium methoxide (2.03 g, 28.9 mmol) and eugenol (67.4 g, 410 mmol). The resulting mixture is heated to 120 °C, at which point tristyrylphenol glycidyl ether ("TSPGE," 183 g, 410 mmol) is added in portions over 22 min. After stirring at 120 °C for 7 hours, ¹H NMR shows complete consumption of the epoxide. The hot reaction mixture is poured into a jar and allowed to cool to room temperature to give the desired secondary alcohol (238 g, 95.1%). The product contains 0.45% K+ by mass.

### EXAMPLE 25

### Hydrophobe from Phenyl Glycidyl Ether and Farnesol

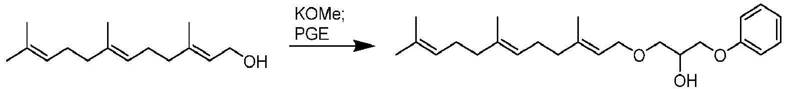

A 500-mL flask is equipped as in Example 17. The addition funnel is charged with phenyl glycidyl ether (101 g, 675 mmol). Under a flow of nitrogen, the flask is charged with solid potassium methoxide (2.05 g, 29.2 mmol) and farnesol (150 g, 674 mmol). The resulting mixture is heated to 110 °C, at which point phenyl glycidyl ether is added from the addition funnel over 26 min. After the addition is complete, the mixture self-heats to 120°C, then cools to 110 °C. After stirring at 110 °C for 18 h, ¹H NMR shows complete consumption of the epoxide. The hot reaction mixture is poured into a jar and allowed to cool to room temperature to give the desired fatty secondary alcohol hydrophobe (249 g, 99.3%). The product contains 0.45% K+ by mass.

### EXAMPLE 26

### Hydrophobe from 1,4-Butanediol Vinyl Ether and Tristyrylphenol Glycidyl Ether

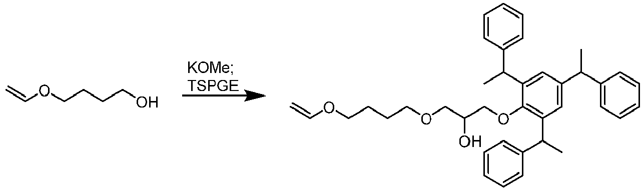

A 500-mL flask is equipped as in Example 17 except that an addition funnel is not included. Under a flow of nitrogen, the flask is charged with solid potassium methoxide (2.04 g, 29.1 mmol) and 1,4-butanediol vinyl ether (51.7 g, 445 mmol). The resulting mixture is heated to 120 °C, at which point tristyrylphenol glycidyl ether (198 g, 444 mmol) is added in portions over 24 min. After stirring at 120 °C for 15.5 hours, ¹H NMR shows complete consumption of the epoxide. The hot reaction mixture is poured into a jar and allowed to cool to room temperature to give the desired secondary alcohol (241 g, 96.4%). The product contains 0.45% K+ by mass.

### EXAMPLE 27

### Hydrophobe from Tristyrylphenol, Phenyl Glycidyl Ether, and Allyl Glycidyl Ether

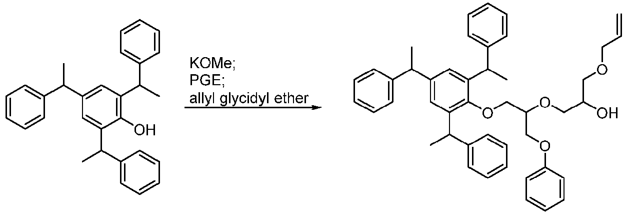

A 500-mL flask is equipped as in Example 17. The addition funnel is charged with phenyl glycidyl ether (57.4 g, 382 mmol). Under a flow of nitrogen, the flask is charged with solid potassium methoxide (2.04 g, 29.1 mmol) and tristyrylphenol (149.2 g, 382 mmol). The resulting mixture is heated to 120 °C, at which point phenyl glycidyl ether is added from the addition funnel over 7 min. After stirring for 3 h and 50 min., an aliquot is removed and analyzed. ¹H NMR shows complete consumption of the epoxide. The addition funnel is removed and replaced with a second addition funnel charged with allyl glycidyl ether (43.6 g, 382 mmol). Allyl glycidyl ether is then added over 13 min. After stirring for 17 h, ¹H NMR shows complete consumption of the second epoxide. The hot reaction mixture is poured into a jar and allowed to cool to room temperature to give the desired secondary fatty alcohol hydrophobe (245.0 g, 97.9%). The product contains 0.45% K+ by mass.

### EXAMPLE 28

### Hydrophobe from Tristyrylphenol, 1,2-Epoxytetradecane, and Allyl Glycidyl Ether

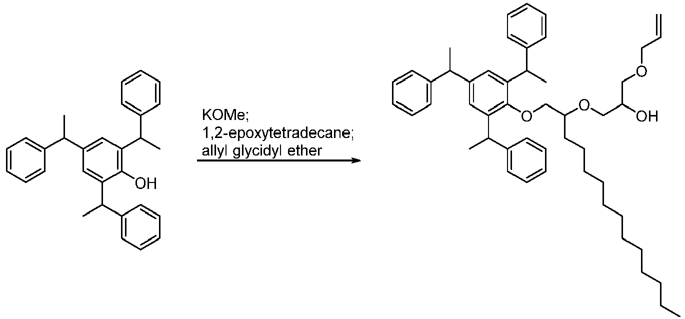

A 500-mL flask is equipped as in Example 17. The addition funnel is charged with 1,2-epoxytetradecane (80.2 g, 378 mmol). Under a flow of nitrogen, the flask is charged with solid potassium methoxide (2.04 g, 29.1 mmol) and tristyrylphenol (147.3 g, 378 mmol). The resulting mixture is heated to 120 °C, at which point 1,2-epoxytetradecane is added from the addition funnel over 7 min. After stirring for 2 h and 30 min., an aliquot is removed and analyzed. ¹H NMR shows 50% consumption of the epoxide. The reaction temperature is increased to 135 °C. After stirring at that temperature for 1 h, ¹H NMR shows 75% consumption of the epoxide. The reaction temperature is increased to 145 °C. After stirring at that temperature for 16 h and 30 min., ¹H NMR shows complete consumption of the epoxide. The addition funnel is removed and replaced with a second addition funnel charged with allyl glycidyl ether (43.1 g, 378 mmol). Allyl glycidyl ether is then added over 15 min. After stirring for 4 h and 45 min., ¹H NMR shows complete consumption of the second epoxide. The hot reaction mixture is poured into a jar and allowed to cool to room temperature to give the desired secondary fatty alcohol hydrophobe (265 g, 98.1%). The product contains 0.42% K+ by mass.

### EXAMPLE 29

### Hydrophobe from Cinnamyl Alcohol and Tristyrylphenol Glycidyl Ether

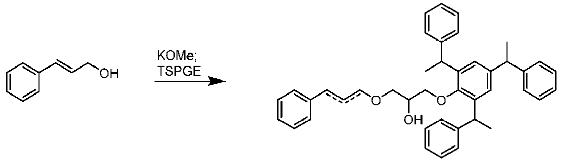

A 500-mL flask is equipped as in Example 17 except that an addition funnel is not included. Under a flow of nitrogen, the flask is charged with solid potassium methoxide (2.04 g, 29.1 mmol) and cinnamyl alcohol (57.8 g, 431 mmol). The resulting mixture is heated to 120 °C, at which point tristyrylphenol glycidyl ether (192.2 g, 431 mmol) is added in portions over 14 min. After stirring at 120 °C for 16h and 15 min., ¹H NMR shows complete consumption of the epoxide. The hot reaction mixture is poured into a jar and allowed to cool to room temperature to give the desired secondary alcohol (244 g, 97.6%) as a mixture of alkene isomers. The product contains 0.45% K+ by mass.

### EXAMPLE 30

### Hydrophobe from Propenyl Styrenated Phenols and Phenyl Glycidyl Ether

### Part 1: Preparation of Styrenated Phenol Allyl Ethers

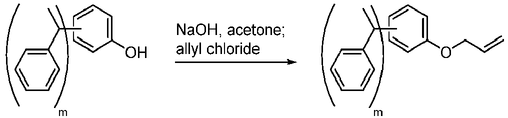

A 500-mL flask is equipped as in Example 17. The addition funnel is charged with allyl chloride (162 g, 2.12 mol). Under a flow of nitrogen, the flask is charged with styrenated phenols (Sanko, 75.2% monostyrenated, 23.5% distyrenated, and 0.7% tristyrenated, 382.5 g, 1.76 mol) and acetone (808 g, 7.89 mol). Solid sodium hydroxide (70.8 g, 1.77 mol) is added in portions over 38 min. During the addition, the reaction temperature increases from 22.0 °C to 25.8 °C. The reaction mixture is slowly heated to 40 °C, at which point allyl chloride is added from the addition funnel over 27 min. After stirring for 17 h and 40 min., an aliquot is removed and analyzed. ¹H NMR shows that the reaction has gone to completion. The mixture is allowed to cool to room temperature and is diluted with acetone (500 g). The mixture is poured into a fritted funnel and the solids are rinsed with acetone (3 x 100 g). The rinses are combined with the filtrate and set aside. The solids are transferred to a 2-L beaker, diluted with acetone (800 g), and stirred at 650 rpm for 30 min. Solids are removed on a fritted funnel, and the filtrates are combined and concentrated by rotary evaporation.

The concentrate is diluted with ethyl acetate (500 mL) and is transferred to a separatory funnel. The container that held the concentrate is rinsed with ethyl acetate (100 mL), and the rinse is added to the same separatory funnel. Hexane (300 mL) is added, and the mixture is sequentially washed with water (2 x 200 mL) and 10% aqueous sodium chloride (250 mL). Solvent is stripped, and residual volatiles are removed under vacuum (1.5 torr) at 70 °C. The product is the expected mixture of styrenated phenol allyl ethers (541.1 g, 74.8%).

### Part 2: Isomerization of Styrenated Phenol Allyl Ethers to Propenyl Styrenated Phenols

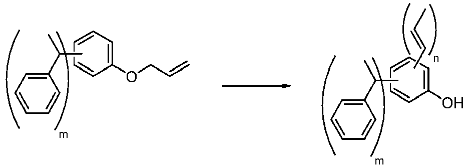

A 1-L resin kettle with INSTATHERM^{®} heating is charged with the styrenated phenol allyl ether mixture prepared above (541 g). The reactor is fitted with an overhead stirrer, a thermocouple with attached temperature controller, and a nitrogen inlet/sparging tube. Nitrogen gas is bubbled through the reaction mixture for 10 min., after which the inlet/sparging tube is removed and the reaction vessel is sealed. The mixture is heated at 200 °C for 5 h and then let cool to 55 °C. The reactor is unsealed and a nitrogen inlet/sparging tube is inserted. ¹H NMR analysis of an aliquot is consistent with the formation of the desired isomerization product. Gas chromatography shows complete conversion of the starting material to propenyl styrenated phenols. The reaction mixture is transferred to a jar (530.5 g, 98.9%).

### Part 3: Hydrophobe Preparation

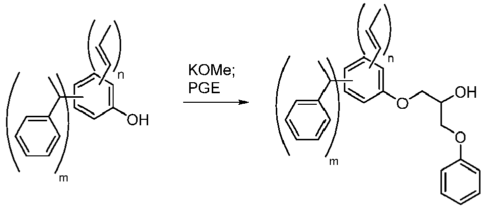

A 500-mL flask is equipped as in Example 17. The addition funnel is charged with phenyl glycidyl ether (92.4 g, 616 mmol). Under a flow of nitrogen, the flask is charged with solid potassium methoxide (2.03 g, 28.9 mmol) and a portion of the propenyl styrenated phenol mixture prepared in Part 2 (158.1 g, 615 mmol). The resulting mixture is heated to 120 °C, at which point phenyl glycidyl ether is added from the addition funnel over 26 min. After stirring for 17 h and 40 min., an aliquot is removed and analyzed. ¹H NMR shows complete consumption of the epoxide. The hot reaction mixture is poured into a jar and allowed to cool to room temperature to give the desired secondary fatty alcohol hydrophobe (244.9 g, 97.8%). The product contains 0.45% K+ by mass.

### EXAMPLE 31

### Hydrophobe from 2-Allylphenol, Resorcinol Diglycidyl Ether, and 1,2-Epoxytetradecane

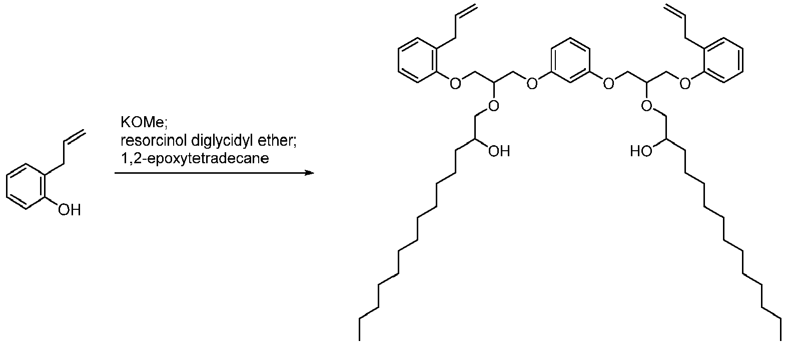

A 500-mL flask is equipped as in Example 17 except that an addition funnel is not included. Under a flow of nitrogen, the flask is charged with solid potassium methoxide (2.21 g, 31.5 mmol) and 2-allylphenol (79.7 g, 594 mmol). The resulting mixture is heated to 110 °C, at which point resorcinol diglycidyl ether (66.2 g, 298 mmol) is added in portions over 12 min. After stirring at 110 °C for 90 min., ¹H NMR shows complete consumption of the epoxide. The reaction temperature is increased to 120 °C. The gas outlet is removed, and an addition funnel charged with 1,2-epoxytetradecane (126.2 g, 594 mmol) is attached to the flask. The gas outlet is then attached to the addition funnel. 1,2-Epoxytetradecane is then added over 26 min. After stirring for 21 h, ¹H NMR shows complete consumption of the second epoxide. The hot reaction mixture is poured into a jar and allowed to cool to room temperature to give the desired secondary fatty alcohol hydrophobe (267 g, 98.1%). The product contains 0.45% K+ by mass.

### EXAMPLE 32

### Hydrophobe from Trimethylolpropane Diallyl Ether and Tristyrylphenol Glycidyl Ether

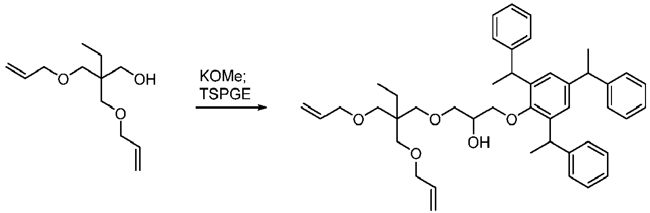

A 500-mL flask is equipped as in Example 17 except that an addition funnel is not included. Under a flow of nitrogen, the flask is charged with solid potassium methoxide (2.03 g, 28.9 mmol) and trimethylolpropane diallyl ether (81.3 g, 380 mmol). The resulting mixture is heated to 120 °C, at which point tristyrylphenol glycidyl ether (169.5 g, 380 mmol) is added in portions over 16 min. After stirring at 120 °C for 21 h and 30 min., ¹H NMR shows complete consumption of the epoxide. The hot reaction mixture is poured into a jar and allowed to cool to room temperature to give the desired secondary alcohol (245 g, 97.9%). The product contains 0.45% K+ by mass.

### EXAMPLE 33

### Hydrophobe from 1-Dodecanethiol, Resorcinol Diglycidyl Ether, and Allyl Glycidyl Ether

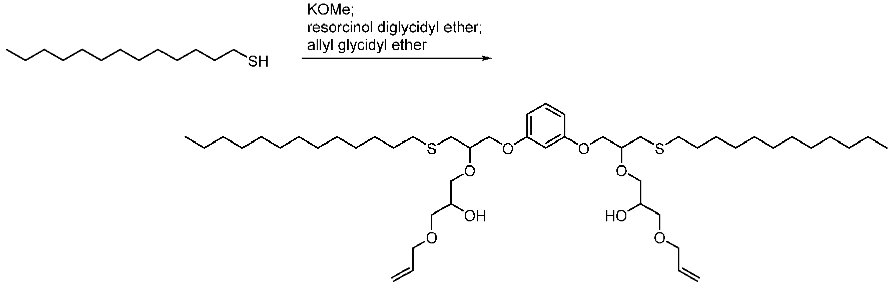

A 500-mL flask is equipped as in Example 17 except that an addition funnel is not included. Under a flow of nitrogen, the flask is charged with solid potassium methoxide (2.03 g, 28.9 mmol) and 1-dodecanethiol (118.4 g, 585 mmol). The resulting mixture is heated to 100 °C, at which point resorcinol diglycidyl ether (65.1 g, 293 mmol) is added in portions over 14 min. After stirring at 100 °C for 45 min., ¹H NMR shows complete consumption of the epoxide. The reaction temperature is increased to 110 °C. The gas outlet is removed, and an addition funnel charged with allyl glycidyl ether (66.8 g, 586 mmol) is attached to the flask. The gas outlet is then attached to the addition funnel. Allyl glycidyl ether is then added over 12 min. After stirring for 5 h and 15 min., ¹H NMR shows complete consumption of the second epoxide. The hot reaction mixture is poured into a jar and allowed to cool to room temperature to give the desired secondary fatty alcohol hydrophobe (246 g, 98.1%). The product contains 0.45% K+ by mass.

### EXAMPLE 34

### Hydrophobe from Allylstyrylphenols, Phenyl Glycidyl Ether, and Allyl Glycidyl Ether

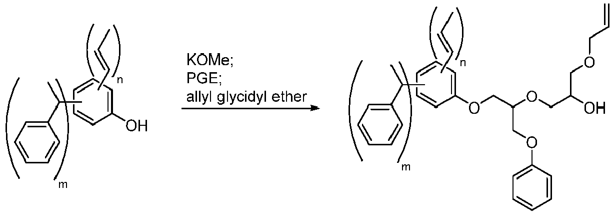

A 500-mL flask is equipped as in Example 17. The addition funnel is charged with phenyl glycidyl ether (72.1 g, 480 mmol). Under a flow of nitrogen, the flask is charged with solid potassium methoxide (2.04 g, 28.9 mmol) and a mixture of allylstyrylphenols prepared as previously described (123.3 g, 480 mmol). The resulting mixture is heated to 120 °C, at which point phenyl glycidyl ether is added from the addition funnel over 11 min. After stirring for 1 h and 50 min., an aliquot is removed and analyzed. ¹H NMR shows complete consumption of the epoxide. The addition funnel is removed and replaced with a second addition funnel charged with allyl glycidyl ether (54.8 g, 480 mmol). Allyl glycidyl ether is then added over 13 min. After stirring for 4 h and 30 min., ¹H NMR shows complete consumption of the second epoxide. The hot reaction mixture is poured into a jar and allowed to cool to room temperature to give the desired secondary fatty alcohol hydrophobe (243 g, 97.1%). The product contains 0.42% K+ by mass.

### EXAMPLE 35

### Hydrophobe from 2-7-Octadien-1-ol and Tristyrylphenol Glycidyl Ether

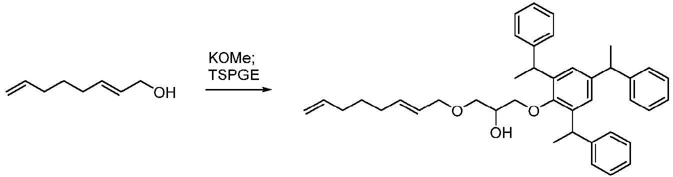

A 500-mL flask is equipped as in Example 17 except that an addition funnel is not included. Under a flow of nitrogen, the flask is charged with solid potassium methoxide (2.03 g, 28.9 mmol) and 2,7-octadien-1-ol (55.1 g, 437 mmol). The resulting mixture is heated to 120 °C, at which point tristyrylphenol glycidyl ether (194.8 g, 437 mmol) is added in portions over 14 min. After stirring at 120 °C for 21 h and 15 min., ¹H NMR shows complete consumption of the epoxide. The hot reaction mixture is poured into a jar and allowed to cool to room temperature to give the desired secondary alcohol (238 g, 95.3%). The product contains 0.45% K+ by mass.

### EXAMPLE 36

### Hydrophobe from Phytol and Phenyl Glycidyl Ether

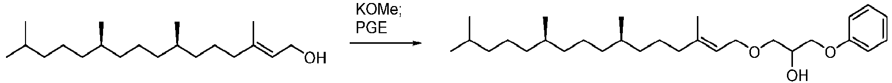

A 500-mL flask is equipped as in Example 17. The addition funnel is charged with phenyl glycidyl ether (77.2 g, 514 mmol). Under a flow of nitrogen, the flask is charged with solid potassium methoxide (1.83 g, 26.1 mmol) and phytol (152.2 g, 513 mmol). The resulting mixture is heated to 110 °C, at which point phenyl glycidyl ether is added from the addition funnel over 20 min. After stirring for 17 h and 45 min., an aliquot is removed and analyzed. ¹H NMR shows complete consumption of the epoxide. The hot reaction mixture is poured into a jar and allowed to cool to room temperature to give the desired secondary fatty alcohol hydrophobe (228 g, 99.5%). The product contains 0.45% K+ by mass.

### EXAMPLE 37

### Wet Adhesion-Promoting Reactive Surfactant

A round-bottom flask equipped with agitator, heating mantle, thermocouple, and heat controller is charged a sample of a reaction product of 2-allylphenol, 1,2-epoxytetradecane, and 20 moles of ethylene oxide (153.4 g, 125 mmol, <500 ppm moisture) prepared according to the method of Example 1. The flask is warmed to 35 °C. Succinic anhydride (13.8 g, 138 mmol) is added, and the mixture is heated to 95 °C. After 1 h, FTIR analysis indicates a complete reaction (disappearance of the absorption band for the anhydride carbonyl stretch at 1785 cm⁻¹).

A portion of the succinate ester reaction product (50.0 g, 37.7 mmol) is charged to a round-bottom flask equipped with an agitator and a thermometer. Methylene chloride (40 g) is added, and the mixture is cooled in an ice bath with agitation to 10 °C. 1-(2-Hydroxyethyl)-2-imidazolidinone (5.3 g, 37.7 mmol) and 4-dimethylaminopyridine (0.2 g) are added. N,N-Dicyclohexylcarbodiimide ("DCC," 7.8 g, 37.8 mmol, Aldrich) is added over 5 min. with an exotherm to 16 °C. Solid dicyclohexylurea (DCHU) forms as the reaction proceeds. The ice bath is removed after 20 min., and the mixture is allowed to stir overnight. FTIR analysis thereafter shows the disappearance of an absorbance at 2118 cm⁻¹, indicated a complete reaction. The reaction product is filtered to remove the DCHU by-product and recover a clear, light-yellow filtrate. The filter cake is rinsed with a small amount of methylene chloride. The combined filtrates are placed in a crystallization dish to evaporate the solvent overnight. A clear amber viscous liquid (50 g) is recovered. Acid titration indicates a conversion to ester of >90%. ¹H NMR indicates that the allylic protons are unaffected by the DCC coupling reaction. Cloud point in water: >100 °C.

### EXAMPLE 38

### Wet Adhesion-Promoting Reactive Surfactant

A flask equipped as in Example 27 is charged with 2-dodec-1-yl succinic anhydride (50.3 g, 189 mmol, TCI Japan) and dry, molten methoxy-capped polyethylene glycol (MPEG-750, 141.3 g, 189 mmol, Aldrich). The mixture is heated to 100 °C for 2 h, after which FTIR analysis shows disappearance of the absorption band for the anhydride carbonyl stretch at 1785 cm⁻¹.

A portion of the reaction product (75 g, 73.8 mmol) is charged to a round-bottom flask equipped with an agitator and a thermometer. Methylene chloride (50 g) is added to form a solution. 1-(2-Hydroxyethyl)-2-imidazolidinone (9.6 g, 73.8 mmol) and 4-dimethylaminopyridine (0.4 g) are added, and the mixture is cooled in an ice bath with agitation to 6 °C. Molten N,N-dicyclohexylcarbodiimide (15.2 g, 73.8 mmol) is added over 5 min. with an exotherm to 16 °C. The ice bath is removed, and the mixture is allowed to stir overnight. FTIR shows the disappearance of an absorbance at 2118 cm⁻¹, indicating a complete reaction of the DCC.

The reaction product is filtered to remove the DCHU by-product and recover a clear, light-yellow filtrate. The filter cake is rinsed with a small amount of methylene chloride. The combined filtrates are placed in a crystallization dish and warmed to 35 °C in a stream of air to evaporate the solvent over several hours. Acid titration indicates a conversion to ester of >95%. ¹H NMR indicates that the allylic protons are unaffected by the DCC coupling reaction. Cloud point in water: 93 °C.

### EXAMPLE 39

### Wet Adhesion-Promoting Reactive Surfactant

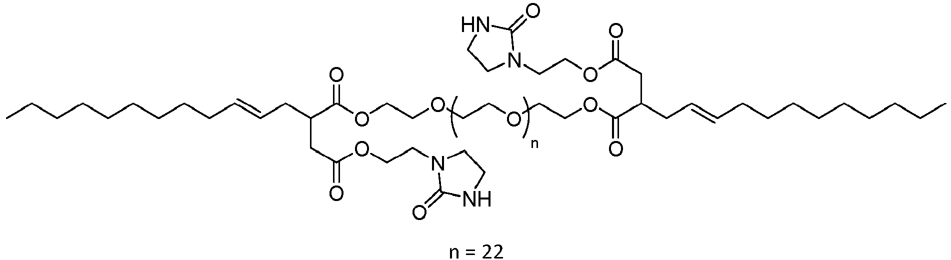

A round-bottom flask equipped with an agitator, a heating mantle, a thermocouple, and a heat controller is charged with 2-dodecyl-1-yl succinic anhydride (82.8 g, 311 mmol, TCI Japan) and dry PEG-1000 (154.9 g, 155 mmol, Aldrich). The reaction mixture is maintained at 100 °C for 8 h, after which FTIR analysis shows disappearance of the absorption band for the anhydride carbonyl stretch at 1785 cm⁻¹.

A portion of the reaction product (128.5 g, 84 mmol) is charged to a round-bottom flask equipped with an agitator and a thermometer. Dichloromethane (150 g) is added to form a solution. Dry 1-(2-hydroxyethyl)-2-imidazolidinone (21.8 g, 168 mmol) and 4-dimethylaminopyridine (0.6 g) are added, and the mixture is cooled in an ice bath with agitation to 6 °C. Molten N,N-dicyclohexylcarbodiimide (34.8 g, 168 mmol, Aldrich) is added over 5 min. with an exotherm to 25 °C. The ice bath is removed, and the mixture is allowed to stir overnight. FTIR analysis shows the disappearance of absorbance at 2118 cm⁻¹ indicating complete conversion of DCC.

The reaction product is filtered to remove the DCHU by-product resulting in a clear, light-yellow filtrate. The filter cake is rinsed with a small amount of dichloromethane. The filtrate is placed in a crystallization dish, and solvent is allowed to evaporate over several days. Acid titration indicates conversion to ester >92%. ¹H NMR indicates that the allylic protons are unaffected by the DCC coupling reaction.

### EXAMPLE 40

### Extractability Studies

Conventional reactive surfactants can contain a significant proportion of non-reactive surfactant. In some cases, the non-reactive surfactant component can interfere with film-forming or produce defects in a coating surface when it migrates to the coating surface. Many of the inventive reactive surfactants will not have significant levels of non-reactive components and should avoid this problem.

To test this idea, the extractability of a reactive surfactant of the invention (an ammonium ether sulfate made from 2-allylphenol, 1,2-epoxytetradecane, 10 moles of EO/mole of hydrophobe, and sulfamic acid ("Reactive Surfactant A")) is compared with that of a commercially available propenyl-substituted nonylphenol 10EO ethoxylate ammonium sulfate ("Comparative Reactive Surfactant B"). A non-reactive "control" example of nonylphenol 10EO ethoxylate ammonium sulfate is included in the study.

Latexes are prepared as generally described above in Example 6L using either Reactive Surfactant A or Comparative Reactive Surfactant B. Latex films are cast on glass plates and dried at 60°C. Films are scraped from the glass with a razor blade. A known mass of latex film (about 1 g) is combined with a known mass of water (about 10 g). The tubes are rotated mechanically at 15 rpm for 24 h.

The concentration of surfactant extracted into the water phase is determined by liquid chromatography. A similar "control" sample having a known amount of nonylphenol 10EO ethoxylate ammonium sulfate is also evaluated using the technique.

The aqueous sample from the latex made using Reactive Surfactant A shows no detectable surfactant in the sample. In contrast, the aqueous sample from the latex made using Comparative Reactive Surfactant B shows a peak corresponding in retention time to the control sample.

This result shows that a portion of Comparative Reactive Surfactant B is 4-nonylphenol 10EO ethoxylate ammonium sulfate, a non-reactive surfactant. This component will be present when 4-nonylphenol is incompletely converted to the corresponding O-allyl ether. Because it is generally impractical to separate unreacted 4-nonylphenol from the O-allyl ether, the unreacted 4-nonylphenol starting material carries as a spectator through the subsequent isomerization step and is then ethoxylated and sulfated. Consequently, the ultimate "reactive" surfactant product will have some proportion of the non-reactive component.

In contrast, 2-allylphenol is relatively easy to separate from phenol by vacuum distillation, so it can be isolated in pure form *before* it is converted to a hydrophobe, ethoxylated, and sulfated. As a result, this "reactive" surfactant will have fewer dead ends. The absence of non-reactive surfactant components should translate into coatings with fewer defects as a result of a reduced degree of surfactant migration to the coating surface during film formation.

Additional extractions are performed with latexes made from the following reactive surfactants using a protocol similar to that described above. In each case, the amount of surfactant extractable from the latex is not detectable.

| Surfactant example | Description |
|---|---|
| 3S | Eugenol-[1,2-epoxytetradecane(1)-EO(16)-SO₃NH₄(1)] |
| 8S | TSP-[PGE(1)-allyl glycidyl ether(1)-EO(15)- SO₃NH₄(1)] |

### Applications Testing

Hydrophobes prepared as described above and identified below in Table 1 are ethoxylated according to the procedure of Example 1; most are subsequently sulfated using the procedure of Example 2. Table 1 summarizes the properties of these surfactants.

| Table 1. Properties of Surfactants from Hydrophobes | | | | |
|---|---|---|---|---|
| Surfactant Example | Hydrophobe from Ex. | Degree of Ethoxylation (mol) | Wt.% solids (50°C, 2 h, in vacuo) | pH (as is) |
| 1S* | 17 | 18 EO | -- | -- |
| 2S* | 18 | 18 EO | -- | -- |
| 3S | 19 | 16 EO | 19.4 | 8.0 |
| 4S | 33 | 12 EO | 21.8 | 8.9 |
| 5S | 21 | 12 EO | 20.3 | 8.7 |
| 6S | 23 | 15 EO | 24.6 | 8.2 |
| 7S | 25 | 17 EO | 24.0 | 8.7 |
| 8S | 27 | 15 EO | 24.4 | 8.7 |
| 9S | 28 | 15 EO | 26.9 | 9.0 |
| 10S | 30 | 16 EO | 17.1 | 8.9 |
| EO = ethylene oxide; * Ethoxylate not sulfated. | | | | |

Latexes are prepared from the sulfated surfactants described in Table 1 using the procedures of Comparative Examples 5L and 7L. Table 2 summarizes properties of the latexes. The comparative examples, described previously, are included in the table.

| Table 2. Latexes from Reactive Surfactants | | | | | |
|---|---|---|---|---|---|
| Latex Example | Surfactant Example | Wt.% Surfactant Active on Monomer | Average Particle Size, nm, peak | Coagulum, g | Final Reaction pH |
| A | 3S | 2.0 | 147 | 0.30 | 5.8 |
| B | 4S | 2.0 | 136 | 0.38 | 5.4 |
| C | 5S | 2.0 | 125 | 0.49 | 5.5 |
| D | 6S | 2.0 | 146 | 0.64 | 5.6 |
| E | 7S | 2.0 | 147 | 0.64 | 5.5 |
| F | 8S | 2.0 | 157 | 0.14 | 5.7 |
| G | 9S | 2.0 | 160 | 4.95 | 5.8 |
| H | 10S | 2.0 | 160 | 0.21 | 5.7 |
| 5L | Conventional | 2.0 | 119 | 0.05 | 5.4 |
| 7L | Comparative Reactive | 2.0 | 109 | 0.02 | 5.3 |

### Water-Resistance of Latex Films

Table 3 summarizes the water resistance films made from the latex examples described in Table 2. The films are evaluated for water resistance according to protocols identified as "WR1" or "WR2."

For protocol WR1, latexes having a pH of 8.6-8.7 are dosed with coalescing solvent (TEXANOL^{™} ester alcohol, 5% on latex solids). Samples are drawn down as clear, wet films on black Leneta panels using a #70 wire-wound rod. The films are cured at room temperature for 3 days. The coated panels are then submerged in room temperature deionized water for 4 days.

For protocol WR2, latexes having a pH of 8.7-8.9 are dosed with the same coalescing solvent and drawn down as described for WR1. The films are cured at room temperature for 24 h, then 30 min. at 50 °C, then cooled to room temperature. The coated panels are then submerged in room temperature deionized water for 116 h.

The coated panels produced by either protocol are evaluated for blushing. Appearance is noted in Table 3 according to a film's tendency to blush. Blushing is rated as "slight," "medium," or "severe." An opaque milky blue hue indicates slight blushing, while a solid white color indicates severe blushing. A film appearance between solid white and milky blue indicates medium blushing.

| Table 3. Water Resistance Testing of Latex Films | | |
|---|---|---|
| Latex Example | Protocol | Blush Ranking |
| | | |
| A | WR1 | slight\medium |
| D | WR1 | slight |
| F | WR1 | medium |
| 5L | WR1 | severe |
| 7L | WR1 | medium |
| | | |
| B | WR2 | medium |
| C | WR2 | slight |
| E | WR2 | medium |
| G | WR2 | medium |
| H | WR2 | medium |
| 5L | WR2 | severe |
| 7L | WR2 | medium |

Foaming properties of the latex examples from Table 2 are evaluated using the following protocol. Low foaming is desirable for latex coatings. A sample (10 g) of each latex is added to a 20-mL scintillation vial and is shaken by hand for 1 min. Foaming is rated as "low" (0 to 3 mL), "medium" (4 to 6 mL), or "high" (7-10 mL). Table 4 summarizes the results.

| Table 4. Foam Ratings of Latexes | |
|---|---|
| Latex Example | Foam Rating_ |
| A | low |
| B | low |
| C | low |
| D | low |
| E | medium |
| F | low |
| G | low |
| H | medium |
| 5L | high |
| 7L | high |

Contact angles of films from the latex examples in Table 2 are measured according to the following protocol. Latexes having a pH of 8.7-8.9 are dosed with coalescing solvent (TEXANOL^{™} ester alcohol, 5% on latex solids). Samples are drawn down as clear, wet films on black Leneta panels using 7-mil Dow wet-film applicator. The films are cured at room temperature for 7 days. Contact angles are measured using a Kruss Mobile Surface Analyzer. Droplets (1 microliter) of deionized water are applied to the film and the contact angles are measured after 13 seconds. Five replicates for each film are obtained, and the results are averaged. Results appear in Table 5.

| Table 5. Contact Angle of Latex Films | |
|---|---|
| Latex Example | Contact angle (degrees) |
| A | 58.1 |
| B | 55.6 |
| C | 60.2 |
| D | 60.7 |
| E | 63.4 |
| F | 59.0 |
| G | 55.7 |
| H | 56.8 |
| 5L | 45.5 |
| 7L | 53.5 |

The preceding examples are meant only as illustrations; the following claims define the scope of the invention.

## Claims

1. A reactive surfactant composition comprising an unsaturated compound of the formula:
M-OOC-CH₂CHR-CO₂CH₂CH₂-Z
or
M-OOC-CHRCH₂-CO₂CH₂CH₂-Z
or
M-OOC-CH=CH-COO-CH₂-CH₂-Z
wherein M is hydrogen, ammonium, an alkali metal, or R²(OCH₂CH₂)ₙ-
for which n is 1 to 50 and R² is C₁-C₄ alkyl, or R² is -COCHRCH₂CO₂CH₂CH₂Z, or R² is -COCH₂CHRCO₂CH₂CH₂Z, or R² is -COCH=CHCO₂CH₂CH₂Z; R is a monounsaturated C₈-C₆₄ group; and Z is an N-imidazolidinone group, preferably wherein M is ammonium and R is a C₁₀-C₁₆ group.

2. A polymerizable mixture comprising the composition of claim 1 and an acrylic monomer.

3. The mixture of claim 2 comprising an aqueous latex emulsion.

4. A coating made from the latex emulsion of claim 3.

5. A process for making a reactive surfactant, comprising reacting an alkyl- or alkenylsuccinic anhydride with an olefin-functional nucleophile to produce an olefin-functional succinate monoester, and optionally, neutralizing the resulting monoester.

6. The process of claim 5, wherein the alkenylsuccinic anhydride is made by heating maleic anhydride with an alpha-olefin under conditions effective to promote an ene reaction.

7. The process of claim 5 or claim 6, wherein the olefin-functional nucleophile is selected from the group consisting of hydroxy-functional acrylates, hydroxy-functional acrylamides, alkoxylates thereof, and mixtures thereof.

8. The process of claim 7, wherein the olefin-functional nucleophile is selected from the group consisting of 2-hydroxyethyl acrylate, 2-hydroxypropyl acrylate, 2-hydroxyethyl methacrylate, 2-hydroxypropyl methacrylate, 2-hydroxyethyl acrylamide, 2-hydroxypropyl acrylamide, 2-hydroxyethyl methacrylamide, 2-hydroxypropyl methacrylamide, 3-(acryloyloxy)-2-hydroxypropyl methacrylate, 2-hydroxy-3-phenoxypropyl acrylate, alkoxylates thereof, and mixtures thereof.

9. The process of claim 5 or claim 6, wherein the olefin-functional nucleophile is selected from the group consisting of olefin-functional phenols, olefin-functional polyphenols, olefin-functional alcohols, olefin-functional polyalcohols, alkoxylates thereof, and mixtures thereof.

10. The process of claim 9, wherein the olefin-functional alcohol or olefin-functional polyalcohol is selected from the group consisting of allyl alcohol, methallyl alcohol, ethylene glycol monoallyl ether, cinnamyl alcohol, 2-hydroxy-2-methyl-3-butene, 2-methyl-3-buten-1-ol, 1,4-butanediol monoallyl ether, 1,4-butanediol monovinyl ether, trimethylolpropane diallyl ether, trimethylolpropane allyl ether, triethylolpropane diallyl ether, triethylolpropane allyl ether, cis-3-hexenyl lactate, glycerol α,α'-diallyl ether, 2,7-octadien-1-ol, farnesol, phytol, 5-norbornene-2-methanol, alkoxylates thereof, and mixtures thereof.

11. The process of claim 9, wherein the olefin-functional phenol is selected from the group consisting of eugenol, isoeugenol, propenyl guaethol, trans-ferulic acid, trans-ferulic acid ethyl ester, trans-ferulic acid methyl ester, alkoxylates thereof, and mixtures thereof.

12. A surfactant composition comprising a reactive surfactant made by the process of any of claims 5 to 11.

13. A polymerizable mixture comprising the composition of claim 12 and an acrylic monomer.

14. The mixture of claim 13 comprising an aqueous latex emulsion.

15. A coating made from the latex emulsion of claim 14.
